# EUROPEAN PATENT SPECIFICATION

(11) **EP 2 661 443 B1**
(45) Date of publication and mention of the grant of the patent: **23.09.2015**
(21) Application number: 11700818.5
(22) Date of filing: 06.01.2011
(51) Int. Cl.: C07K 16/10, C07K 14/00

(54) **ALPHABODIES SPECIFICALLY BINDING TO VIRAL PROTEINS AND METHODS FOR PRODUCING THE SAME**
SPEZIFISCH AN VIRALE PROTEINE BINDENDE ALPHAKÖRPER UND VERFAHREN ZU IHRER HERSTELLUNG
ALPHACORPS SE LIANT SPÉCIFIQUEMENT À DES PROTÉINES VIRALES ET LEURS PROCÉDÉS DE PRODUCTION

(43) Date of publication of application: 13.11.2013
(73) Proprietor: Complix SA, 1526 Luxembourg (LU)
(72) Inventor: DESMET, Johan, B-8500 Kortrijk (BE); LASTERS, Ignace, B-2018 Antwerpen (BE); MEERSSEMAN, Geert, B-1000 Brussel (BE); DEROO, Sabrina, F-57330 Roussy le Village (FR)
(74) Representative: Paemen, Liesbet R.J.
(86) International application number: PCT/EP2011/050137
(87) International publication number: WO 2012/092971

(56) References cited:
- EP-A1- 2 161 278
- WO-A1-2009/013352
- WO-A1-2010/066740
- WO-A1-2011/003936
- WO-A2-2005/077103
- WO-A2-2009/030780
- J. LOUIS ET AL.: "Covalent trimers of the internal N-terminal trimeric coiled-coil of gp41 and antibodies directed against them are potent inhibitors of HIV envelope-mediated cell fusion.", THE JOURNAL OF BIOLOGICAL CHEMISTRY, vol. 278, no. 22, 30 May 2003 (2003-05-30) , pages 20278-20285, XP002481442, U.S.A.
- M. ROOT ET AL.: "Protein design of an HIV-1 entry inhibitor.", SCIENCE, vol. 291, 2 February 2001 (2001-02-02), pages 884-888, XP002170634,

## Description

### FIELD OF THE INVENTION

The present invention relates to the field of binding agents directed against viral proteins and methods for producing such binding agents as well as uses of such binding agents for prophylactic, therapeutic or diagnostic purposes.

### BACKGROUND

One of the essential steps in viral infection is the fusion between the virus membrane and the membrane of the host cell. Viral infection is mediated by viral glycoproteins, including viral attachment proteins and fusion-driving viral fusion proteins. Viral attachment and fusion-driving proteins are collectively referred to herein as viral fusion proteins. Viral membrane fusion with the host cell can take place either at the plasma membrane or at an intracellular location (endosome) following virus uptake by endocytosis (Earp et al. Curr. Topics Microbiol. Immunol. 285: 25-66 (2005); Smith et al. Science 304: 237-242 (2004)).

Antibody therapy using polyclonal and monoclonal antibodies (mAbs) has been effective in prophylaxis of varicella, hepatitis A, hepatitis B, rabies (Montano-Hirose et al., Vaccine 11: 1259-1266 (1993); Schumacher et al., Vaccine 10: 754-760 (1992)), and respiratory syncytial virus infections (Sawyer, Antiviral Res. 47: 57-77 (2000)). In the past decade, two antibodies have been licensed for a viral indication, namely RSV-IG (i.e., RespiGam) and Palivizumab (i.e., Synagis®), both for prevention of respiratory syncytial virus infection. Cytogam^{®} is indicated for the prophylaxis of cytomegalovirus disease associated with transplantation of kidney, lung, liver, pancreas, and heart. Antibody-based therapy for human patients with influenza is up to now little explored. Nevertheless, it has been demonstrated that specific monoclonal antibodies can confer prophylactic and therapeutic protection against influenza in mice (Smirnov et al., Arch. Virol. 145: 1733-1741 (2000); Renegar et al., J. Immunol. 173: 1978-1986 (2004); Palladino et al., J. Virol. 69: 2075- 2081 (1995)). Humanized mouse mAbs and equine F(ab')2 fragments specific for hemagglutinin H5 protein of the influenza virus have also been used for prophylaxis and therapy in a mouse model (Lu et al., Respir. Res. 7: 43 (2006); Hanson et al,. Respir. Res. 7: 126 (2006)). Antibody fragments, such as F(ab')2 fragments, Fab fragments (Lamarre et al. J. Immunol. 154: 3975-3984 (1995); Thullier et al., J. Biotechnol. 69: 183-190 (1999); Schofield et al., J. Gen. Virol. 78: 2431-2439 (1997); Barbas et al. Proc. Natl. Acad. Sci. USA 89: 10164 (1992); Crowe et al., Proc. Natl. Acad. Sci. USA 91: 1386 (1994); Prince et al., J. Virol. 64: 3091 (1990)), single-chain Fv fragments (Mason et al. Virology 224: 548 (1996)) and variable domains derived from camelid species heavy chain antibodies (Sherwood et al., J. Infect. Dis. 196: S213-219 (2007); Dekker et al., J. Virol. 11: 12132-12139 (2003); Goldman et al., Anal. Chem. 78: 8245-8255 (2006)) have also proven to be successful in neutralizing a variety of enveloped viruses both in vitro and in vivo in animal models (predominantly in mice).

J. Louis et al. (J. Biol. Chem. 278(22):20278-20285, 2003) disclose that covalent trimers of the internal N-terminal trimeric coiled-coil of gp41 are potent inhibitors of HIV envelope-mediated cell fusion. WO2005/077103 discloses that peptides derived from the HR-N and HR-C regions of the coronavirus SARS S-protein each have a stable alpha-helical coiled coil trimeric conformation and allege that these peptides inhibit viral activity.

Nevertheless, the development of effective and potent antiviral drugs remains a major scientific challenge. Only for a minority of viral infections, there is at present an effective prophylactic and/or therapeutic compound available. In addition, the antiviral drugs that are currently on the market show numerous side-effects, such as nausea, vomiting, skin rashes, migraine, fatigue, trembling, and, more rarely, epileptic seizures. Also, the constant ability of viruses to mutate and adapt themselves to the environmental conditions, such as challenges by neutralizing antibodies or neutralizing therapeutic compounds, presents an enormous difficulty to the design of antiviral strategies that are effective over the long term.

Accordingly, there remains a serious need for new potent antiviral drugs for the treatment and prevention of infectious viral diseases as well as for alternative and improved antiviral drugs that are more efficient, preferably over the long term, in comparison with the existing antiviral agents that are currently on the market.

WO2009/030780 discloses non-natural proteinaceous scaffold made of 3 non-covalently associated peptides, which can be mutated to bind specific target molecules. It is suggested to use these peptides to block HIV viral entry. WO 2010/066740 and EP 2 161 278 describe Alphabody scaffolds as single-chain triple-stranded alpha-helical coiled coil scaffolds. Both applications describe the architecture and physico-chemical properties of such scaffolds. WO 2010/066740 provides single-chain Alphabody scaffolds which adopt a so-called antiparallel structure, i.e., an architecture wherein one of the three alpha-helices in a single-chain Alphabody is oriented antiparallel with respect to the other two alpha-helices; the three alpha-helices thus constitute an antiparallel coiled coil structure. In contrast, EP 2 161 278 A1 provides single-chain Alphabody scaffolds which adopt an all-parallel structure, i.e., an architecture wherein all three alpha-helices in a single-chain Alphabody together form a parallel coiled coil structure. However, it has not been disclosed how these Alphabody scaffolds can be manipulated to obtain Alphabodies specifically binding to targets of interest.

### SUMMARY OF THE INVENTION

The present inventors have developed new methods which allow the generation of Alphabodies which specifically bind to a viral target protein or peptide of interest. It has been found that using the Alphabody scaffold, binders can be generated which bind to viral target of interest with high affinity and specificity and which overcome one or more of the disadvantages of the prior art binders. Moreover it has been found that such binders have several advantages over the traditional (immunoglobulin and non-immunoglobulin) binding agents known in the art. Such advantages include, without limitation, the fact that they are compact and small in size (between 10 and 14 kDa, which is 10 times smaller than an antibody), they are extremely (thermo)stable (having a melting temperature of more than 100 °C), and are relatively insensitive to changes in pH and to proteolytic degradation. In addition, Alphabodies are highly soluble, they are highly engineerable (in the sense that multiple substitutions will generally not obliterate their correct and stable folding), and have a structure which is based on natural motifs but is designed via protein engineering techniques.

In one aspect, the present invention provides methods for the production of at least one single-chain Alphabody having detectable binding affinity for, or detectable in vitro activity on, a viral protein of interest, the method at least comprising the steps of:
a) producing a single-chain Alphabody library comprising at least 100 different-sequence single-chain Alphabody polypeptides, wherein the Alphabody polypeptides differ from each other in at least one of a defined set of 5 to 20 variegated amino acid residue positions, and wherein at least 70% of the variegated amino acid residue positions are located either:
   (i) at heptad e-positions in a first alpha-helix of the Alphabody polypeptides and at heptad g-positions in a second alpha-helix, and optionally at heptad b-positions in the first alpha-helix of the Alphabody polypeptides and/or at heptad c-positions in the second alpha-helix of the Alphabody polypeptides, or
   (ii) at heptad b-, c- and f-positions in one alpha-helix of the Alphabody polypeptides, or
   (iii) at positions in a linker fragment connecting two consecutive alpha-helices of the Alphabody polypeptides; and
b) selecting from the single-chain Alphabody library at least one single-chain Alphabody having detectable binding affinity for, or detectable in vitro activity on, the viral protein of interest.

Thus in the methods of the present invention the variegated amino acid residue positions specified in step (a) are located for at least 70% in one of the regions referred to herein as groove, helix surface or linker fragment regions.

In further particular embodiments of these methods of the invention, step a) of producing a single-chain Alphabody library may comprise the steps of:
a1) producing a nucleic acid or vector library encoding a single-chain Alphabody library comprising at least 100 different-sequence single-chain Alphabody polypeptides, wherein the Alphabody polypeptides differ from each other in at least one of a defined set of 5 to 20 variegated amino acid residue positions, and wherein at least 70% of the variegated amino acid residue positions are located either:
   (i) at heptad e-positions in a first alpha-helix of the Alphabody polypeptides and at heptad g-positions in a second alpha-helix, and optionally at heptad b-positions in the first alpha-helix of the Alphabody polypeptides and/or at heptad c-positions in the second alpha-helix of the Alphabody polypeptides, or
   (ii) at heptad b-, c- and f-positions in one alpha-helix of the Alphabody polypeptides, or
   (iii) at positions in a linker fragment connecting two consecutive alpha-helices of the Alphabody polypeptides, and
a2) expressing the nucleic acid or vector library under conditions suitable for the production of the single-chain Alphabody library, such as for example by introducing the nucleic acid or vector library into host cells and culturing the host cells in a medium under conditions suitable for the production of the single-chain Alphabody library.

In certain particular embodiments, the methods of the present invention may further comprise the step of isolating the single-chain Alphabody library produced by expressing the nucleic acid or vector library under suitable conditions from the host cells and/or from the medium.

In further particular embodiments of the methods of the invention, step b) of selecting from the single-chain Alphabody library at least one single-chain Alphabody having detectable binding affinity for, or detectable in vitro activity on, the viral protein of interest, may comprise contacting the viral protein of interest with the single-chain Alphabody library produced in step a).

Also, in yet further particular embodiments, the methods of the present invention may further comprise the step of isolating from the single-chain Alphabody library at least one single-chain Alphabody having detectable binding affinity for, or detectable in vitro activity on, the viral protein of interest.

In certain embodiments of the methods of the present invention, the produced single-chain Alphabody library may be enriched for single-chain Alphabodies having detectable binding affinity for, or detectable in vitro activity on, the viral protein of interest. This may for example be done by iterative execution of step b) of selecting from the single-chain Alphabody library single-chain Alphabodies having detectable binding affinity for, or detectable in vitro activity on, the viral protein of interest. More particularly, an Alphabody library may be enriched by iterative execution of contacting the viral protein of interest with the said single-chain Alphabody library and isolating from this library single-chain Alphabodies having detectable binding affinity for, or detectable in vitro activity on, the viral protein of interest; it is herein understood that, in the first iteration cycle, the said Alphabody library is the single-chain Alphabody library produced in step a), whereas in each consecutive iteration cycle, the said Alphabody library consists of the single-chain Alphabodies isolated at the end of the previous iteration cycle. In the context of a phage-display method, each such iteration cycle is known in the art as a biopanning round.

In further particular embodiments, the methods of the present invention may further comprise the step of amplifying (multiplying, augmenting the number of) the single-chain Alphabodies obtained after (each) selection step b) wherein single-chain Alphabodies are selected that have detectable binding affinity for, or detectable in vitro activity on, the viral protein of interest.

In particular embodiments of the invention, the viral protein is a viral fusion protein. Viral fusion proteins of interest include any viral surface-displayed protein, such as but not limited to the gp120 protein of HIV-1 virus, the HA1 and HA2 proteins of influenza, the F protein of respiratory syncytial virus, the HA protein of Influenza A virus, the HEF protein of influenza C virus, the F protein of Simian parainfluenza virus, the F protein of Human parainfluenza virus, the F protein of Newcastle disease virus, the F2 protein of measles, the F2 protein of Sendai virus, the gp2 protein of Ebola virus, the TM protein of Moloney murine leukemia virus, the gp41 protein of HIV-1, the gp41 protein of Simian immunodeficiency virus, the gp21 protein of Human T-cell leukemia virus 1, the TM protein of Human syncytin-2, the TM protein of Visna virus, the S2 protein of Mouse hepatitis virus, the E2 protein of SARS corona virus, the E protein of Tick-borne encephalitis virus, the E2 protein of Dengue 2 and 3 virus, the E protein of Yellow Fever virus, the E protein of West Nile virus, the E1 protein of Semliki forest virus, the E1 protein of Sindbis virus, the G protein of Rabies virus, the G protein of Vesicular stomatitis virus, the gB protein of Herpes simplex virus.

In particular embodiments, the methods of the invention provide single-chain Alphabodies having detectable binding affinity for a domain of a viral protein of interest, the latter protein which can be provided as a functionally relevant viral protein domain; non-limiting examples of functionally relevant viral protein domains include a receptor-binding domain, an ectodomain, an attachment domain, a fusion-driving domain, a stably folded subdomain, a domain resulting from proteolytic degradation.

In further particular embodiments, the methods of the present invention provide single-chain Alphabodies having detectable binding affinity for a subregion within a viral protein domain of interest. These include, more particularly functionally relevant subregion of domains; non-limiting examples of functionally relevant subregions within a viral protein domain include a fusion peptide, an N-terminal heptad repeat (N-HR) fragment, a C-terminal heptad repeat (C-HR), a non-heptad repeat fragment which binds to a heptad repeat fragment in the postfusion state of a viral protein of interest, or, in general, any subregion of a viral protein which plays an active, fusion-driving role in the process of membrane fusion with a target cell.

The application discloses single-chain Alphabody polypeptides having detectable binding affinity for, or detectable in vitro activity on, a viral protein of interest, which are obtainable by the methods according to the present invention.

### DETAILED DESCRIPTION OF THE INVENTION

As used herein, the singular forms 'a', 'an', and 'the' include both singular and plural referents unless the context clearly dictates otherwise.

The terms 'comprising', 'comprises' and 'comprised of as used herein are synonymous with 'including', 'includes' or 'containing', 'contains', and are inclusive or openended and do not exclude additional, non-recited members, elements or method steps.

The recitation of numerical ranges by endpoints includes all numbers and fractions subsumed within the respective ranges, as well as the recited endpoints.

The term 'about' as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier 'about' refers is itself also specifically, and preferably, disclosed.

As used herein, an 'Alphabody' or 'Alphabodies ' can generally be defined as self-folded, single-chain, triple-stranded, predominantly alpha-helical, coiled coil amino acid sequences, polypeptides or proteins. The term 'single-chain' in 'single-chain Alphabody' is therefore redundant, but usually included to emphasize the composition of an Alphabody as a single polypeptide chain, as opposed to the many known occurrences of oligomeric (e.g., trimeric) peptidic coiled coils. More particularly, Alphabodies as used in the context of the present invention can be defined as amino acid sequences, polypeptides or proteins having the general formula HRS1-L1-HRS2-L2-HRS3, wherein
- each of HRS1, HRS2 and HRS3 is independently a heptad repeat sequence (HRS) consisting of 2 to 7 consecutive heptad repeat units, at least 50% of all heptad a- and d-positions are occupied by isoleucine residues, each HRS starts and ends with an aliphatic or aromatic amino acid residue located at either a heptad a- or d-position, and HRS1, HRS2 and HRS3 together form a triple-stranded, alpha-helical, coiled coil structure; and
- each of L1 and L2 are independently a linker fragment, covalently connecting HRS1 to HRS2 and HRS2 to HRS3, respectively, and consisting of at least 4 amino acid residues, preferably at least 50% of which are selected from the group proline, glycine, serine.

As used herein, a 'parallel Alphabody' shall have the meaning of an Alphabody wherein the alpha-helices of the triple-stranded, alpha-helical, coiled coil structure together form a parallel coiled coil structure, i.e., a coiled coil wherein all three alpha-helices are parallel.

As used herein, an 'antiparallel Alphabody' shall have the meaning of an Alphabody wherein the alpha-helices of the triple-stranded, alpha-helical, coiled coil structure together form an antiparallel coiled coil structure, i.e., a coiled coil wherein two alpha-helices are parallel and the third alpha-helix is antiparallel with respect to these two helices.

As will become clear from the further description, herein Alphabodies having the general formula HRS1-L1-HRS2-L2-HRS3 may in certain particular embodiments comprise further groups, moieties and/or residues, which are covalently linked, more particularly N-and/or C-terminal covalently linked, to the basic Alphabody structure having the formula HRS1-L1-HRS2-L2-HRS3. The present invention thus generally relates to Alphabody polypeptides comprising one or more Alphabodies according to the invention and/or other groups, moieties and/or residues linked thereto.

The terms 'heptad', 'heptad unit' or 'heptad repeat unit' are used interchangeably herein and shall herein have the meaning of a 7-residue (poly)peptide fragment that is repeated two or more times within each heptad repeat sequence of an Alphabody, polypeptide or composition and is represented as 'abcdefg' or 'defgabc', wherein the symbols 'a' to 'g' denote conventional heptad positions. Conventional heptad positions are assigned to specific amino acid residues within a heptad, a heptad unit, or a heptad repeat unit, present in an Alphabody, polypeptide or composition, for example, by using specialized software such as the COILS method of Lupas et al. (Lupas et al., Science 252:1162-1164 (1994)); http://www.russell.embl-heidelberg.de/cgi-bin/coils-svr.pl). However, it is noted that the heptads or heptad units as present in the Alphabodies (or polypeptides and compositions comprising these Alphabodies) are not strictly limited to the above-cited representations (i.e. 'abcdefg' or 'defgabc') as will become clear from the further description herein and in their broadest sense constitute a 7-residue (poly)peptide fragment per se, comprising at least assignable heptad positions a and d.

The terms 'heptad a-positions', 'heptad b-positions', 'heptad c-positions', 'heptad d-positions', 'heptad e-positions', 'heptad f-positions' and 'heptad g-positions' refer respectively to the conventional 'a', 'b', 'c', 'd', 'e', 'f and 'g' amino acid positions in a heptad, heptad repeat or heptad repeat unit of an Alphabody, polypeptide or composition.

A 'heptad motif as used herein shall have the meaning of a 7-residue (poly)peptide pattern. A 'heptad motif of the type 'abcdefg' can usually be represented as 'HPPHPPP', whereas a 'heptad motif of the type 'defgabc' can usually represented as 'HPPPHPP', wherein the symbol 'H' denotes an apolar or hydrophobic amino acid residue and the symbol 'P' denotes a polar or hydrophilic amino acid residue. However, it is noted that the heptad motifs as present in the Alphabodies disclosed herein (or polypeptides and compositions disclosed herein comprising these Alphabodies) are not strictly limited to the above-cited representations (i.e. 'abcdefg', 'HPPHPPP', 'defgabc' and 'HPPPHPP') as will become clear from the further description herein.

A 'heptad repeat sequence' ('HRS') as used herein shall have the meaning of an amino acid sequence or sequence fragment consisting of n consecutive heptads, where n is a number equal to or greater than 2.

In the context of the single-chain structure of the Alphabodies (as defined herein) the terms 'linker', 'linker fragment' or 'linker sequence' are used interchangeably herein and refer to an amino acid sequence fragment that is part of the contiguous amino acid sequence of a single-chain (monomeric) Alphabody, and covalently interconnects the HRS sequences of that Alphabody.

In the context of the present invention, a 'coiled coil' or 'coiled coil structure' shall be used interchangeably herein and will be clear to the person skilled in the art based on the common general knowledge and the description and further references cited herein: Particular reference in this regard is made to review papers concerning coiled coil structures (such as for example, Cohen and Parry, Proteins 7:1-15 (1990); Kohn and Hodges, Trends Biotechnol. 16:379-389 (1998); Schneider et al., Fold. Des. 8, 3:R29-R40 (1998); Harbury et al., Science 282:1462-1467 (1998); Mason and Arndt, Chem. BioChem. 5:170-176 (2004); Lupas and Gruber, Adv. Protein Chem. 70:37-78 (2005); Woolfson, Adv. Protein Chem. 70:79-112 (2005); Parry et al., J. Struct. Biol. 163:258-269 (2008); McFarlane et al., Eur. J. Pharmacol. 625:101-107 (2009)).

An 'alpha-helical part of an Alphabody' shall herein have the meaning of that part of an Alphabody which has an alpha-helical secondary structure. Furthermore, any part of the full part of an Alphabody having an alpha-helical secondary structure is also considered an alpha-helical part of an Alphabody. More particularly, in the context of a binding site, where one or more amino acids located in an alpha-helical part of the Alphabody contribute to the binding site, the binding site is considered to be formed by an alpha-helical part of the Alphabody.

A 'solvent-oriented' or 'solvent-exposed' region of an alpha-helix of an Alphabody shall herein have the meaning of that part on an Alphabody which is directly exposed or which comes directly into contact with the solvent, environment, surroundings or milieu in which it is present. Furthermore, any part of the full part of an Alphabody which is directly exposed or which comes directly into contact with the solvent is also considered a solvent-oriented or solvent-exposed region of an Alphabody. More particularly, in the context of a binding site, where one or more amino acids located in a solvent-oriented part of the Alphabody contribute to the binding site, the binding site is considered to be formed by a solvent-oriented part of the Alphabody.

The term 'groove of an Alphabody' shall herein have the meaning of that part on an Alphabody which corresponds to the concave, groove-like local shape, which is formed by any pair of spatially adjacent alpha-helices within an Alphabody.

As used herein, amino acid residues will be indicated either by their full name or according to the standard three-letter or one-letter amino acid code.

As used herein, the term 'homology' denotes at least primary structure similarity between two macromolecules, particularly between two polypeptides or polynucleotides, from same or different taxons, wherein said similarity is due to shared ancestry. Preferably, homologous polypeptides will also display similarity in secondary or tertiary structure. Hence, the term 'homologues' denotes so-related macromolecules having said primary and optionally, for proteinaceous macromolecules, secondary or tertiary structure similarity. For comparing two or more nucleotide sequences, the '(percentage of) sequence identity' between a first nucleotide sequence and a second nucleotide sequence may be calculated using methods known by the person skilled in the art, e.g. by dividing the number of nucleotides in the first nucleotide sequence that are identical to the nucleotides at the corresponding positions in the second nucleotide sequence by the total number of nucleotides in the first nucleotide sequence and multiplying by 100% or by using a know computer algorithm for sequence alignment such as NCBI Blast. In determining the degree of sequence identity between two Alphabodies, the skilled person may take into account so-called 'conservative' amino acid substitutions, which can generally be described as amino acid substitutions in which an amino acid residue is replaced with another amino acid residue of similar chemical structure and which has little or essentially no influence on the function, activity or other biological properties of the polypeptide. Possible conservative amino acid substitutions will be clear to the person skilled in the art. Two or more Alphabodies, or two or more nucleic acid sequences are said to be 'exactly the same' if they have 100% sequence identity over their entire length.

An Alphabody, polypeptide or composition as disclosed herein is said to 'specifically bind to' a particular target when that Alphabody, polypeptide or composition has affinity for, specificity for and/or is specifically directed against that target (or for at least one part or fragment thereof).

The 'specificity' of an Alphabody, polypeptide or composition as disclosed herein as used herein can be determined based on affinity and/or avidity. The 'affinity' of an Alphabody, polypeptide or composition is represented by the equilibrium constant for the dissociation of the Alphabody, polypeptide or composition and the target protein of interest to which it binds. The lower the KD value, the stronger the binding strength between the Alphabody, polypeptide or composition and the target protein of interest to which it binds. Alternatively, the affinity can also be expressed in terms of the affinity constant (KA), which corresponds to 1/KD. The binding affinity of an Alphabody, polypeptide or composition can be determined in a manner known to the skilled person, depending on the specific target protein of interest.

It is generally known in the art that the KD can be expressed as the ratio of the dissociation rate constant of a complex, denoted as kOff (expressed in seconds⁻¹ or s⁻¹), to the rate constant of its association, denoted kOn (expressed in molar⁻¹ seconds⁻¹ or M⁻¹ s⁻¹). A KD value greater than about 1 millimolar is generally considered to indicate non-binding or non-specific binding.

The 'avidity' of an Alphabody, polypeptide or composition as disclosed herein is the measure of the strength of binding between an Alphabody, polypeptide or composition and the pertinent target protein of interest. Avidity is related to both the affinity between a binding site on the target protein of interest and a binding site on the Alphabody, polypeptide or composition and the number of pertinent binding sites present on the Alphabody, polypeptide or composition Binding affinities, kOff and kOn rates may be determined by means of methods known to the person skilled in the art. These methods include, but are not limited to RIA (radioimmunoassays), ELISA (enzyme-linked immuno-sorbent assays), 'sandwich' immunoassays, immunoradiometric assays, gel diffusion precipitation reactions, immunodiffusion assays, Western blots, precipitation reactions, agglutination assays (e.g., gel agglutination assays, hemagglutination assays, etc.), complement fixation assays, immunofluorescence assays, immunoelectrophoresis assays, isothermal titration calorimetry, surface plasmon resonance, fluorescence-activated cell sorting analysis, etc.

An Alphabody, polypeptide or composition as disclosed herein is said to be 'specific for a first target protein of interest as opposed to a second target protein of interest' when it binds to the first target protein of interest with an affinity that is at least 5 times, such as at least 10 times, such as at least 100 times, and preferably at least 1000 times higher than the affinity with which that Alphabody, polypeptide or composition binds to the second target protein of interest. Accordingly, in certain embodiments, when an Alphabody, polypeptide or composition is said to be 'specific for' a first target protein of interest as opposed to a second target protein of interest, it may specifically bind to (as defined herein) the first target protein of interest, but not to the second target protein of interest. The same applies when reference is made to specificity for specific protein domains or subregions thereof.

An Alphabody, polypeptide or composition as disclosed herein is said to 'have detectable binding affinity for' a protein of interest, when it binds to that protein of interest (more particularly to a domain or subregion thereof) with an affinity higher than the detection limit of any of the methods known to the person skilled in the art, i.e., methods including but not limited to RIA (radioimmunoassays), ELISA (enzyme-linked immuno-sorbent assays), 'sandwich' immunoassays, immunoradiometric assays, gel diffusion precipitation reactions, immunodiffusion assays, Western blots, precipitation reactions, agglutination assays (e.g., gel agglutination assays, hemagglutination assays, etc.), complement fixation assays, immunofluorescence assays, immunoelectrophoresis assays, isothermal titration calorimetry, surface plasmon resonance, fluorescence-activated cell sorting analysis, etc.

The 'half-life' of an Alphabody, polypeptide or compound can generally be defined as the time that is needed for the in vivo serum or plasma concentration of the Alphabody, polypeptide or compound to be reduced by 50%. The in vivo half-life of an Alphabody, compound or polypeptide can be determined in any manner known to the person skilled in the art, such as by pharmacokinetic analysis. As will be clear to the skilled person, the half-life can be expressed using parameters such as the t1/2-alpha, t1/2-beta and the area under the curve (AUC). An increased half-life in vivo is generally characterized by an increase in one or more and preferably in all three of the parameters t1/2-alpha, t1/2-beta and the area under the curve (AUC).

As used herein, the terms 'inhibiting', 'reducing' and/or 'preventing' may refer to (the use of) an Alphabody, polypeptide or composition according to the invention that specifically binds to a target protein of interest (in particular to a target protein domain or subregion thereof), and inhibits, reduces and/or prevents the interaction between that target protein and its natural binding partner. The terms 'inhibiting', 'reducing' and/or 'preventing' may also refer to (the use of) an Alphabody, polypeptide or composition according to the invention that specifically binds to a target protein and inhibits, reduces and/or prevents a biological activity of that target protein of interest, as measured using a suitable in vitro, cellular or in vivo assay. Accordingly, 'inhibiting', 'reducing' and/or 'preventing' may also refer to (the use of) an Alphabody, polypeptide or composition according to the invention that specifically binds to a target protein of interest and inhibits, reduces and/or prevents one or more biological or physiological mechanisms, effects, responses, functions pathways or activities in which the target protein of interest is involved. Such an action of the Alphabody, polypeptide or composition according to the invention as an antagonist, in the broadest possible sense, may be determined in any suitable manner and/or using any suitable (in vitro and usually cellular or in vivo) assay known in the art, depending on the type of inhibition, reduction and/or prevention of the said one or more biological or physiological mechanisms, effects, responses, functional pathways or activities in which the said target protein is involved. Non-limiting examples of such types of functional effects include (i) the prevention of attachment to cellular receptors on specific, dedicated target cells, (ii) the prevention of interaction with the glycocalyx of target cells, (iii) the formation of a steric block at the surface of a virion or a viral protein-displaying cell, (iv) the arrest of a viral protein in its native state through blockage of conformational changes that are required for membrane fusion, (v) the arrest of a viral protein in a conformational or mechanistic state that is intermediate to the native and postfusion states, (vi) the irreversible functional deactivation of a viral protein prior to attachment to a target cell, and wherein said deactivation is further characterized by the inability of said viral protein to recover membrane fusion activity even after removal of the antiviral Alphabody, polypeptide or composition according to the invention.

In addition, 'inhibiting', 'reducing' and/or 'preventing' may also mean inducing a decrease in affinity, avidity, specificity and/or selectivity of a viral target protein of interest for one or more of its natural binding partners and/or inducing a decrease in the sensitivity of the viral target protein of interest for one or more conditions in the medium or surroundings in which the viral target protein of interest is present (such as pH, ion strength, the presence of co-factors, etc.), compared to the same conditions but without the presence of the Alphabody, polypeptide or composition. In the context of the present invention, 'inhibiting', 'reducing' and/or 'preventing' may also involve allosteric inhibition, reduction and/or prevention of the activity of a viral target protein of interest.

The said 'inhibiting', 'reducing' and/or 'preventing' activity of an Alphabody, polypeptide or composition may be reversible or irreversible.

An Alphabody, polypeptide, composition or nucleic acid sequence is herein considered to be '(in) essentially isolated (form)' when it has been extracted or purified from the host cell and/or medium in which it was produced.

In respect of the Alphabodies, polypeptides and (pharmaceutical) compositions, the terms 'binding region', 'binding site' or 'interaction site' present on the Alphabodies, polypeptides or pharmaceutical compositions shall herein have the meaning of a particular site, part, domain or stretch of amino acid residues present on the Alphabodies, polypeptides or pharmaceutical compositions that is responsible for binding to a target molecule. Such binding region essentially consists of specific amino acid residues from the Alphabody which are in contact with the target molecule, in particular, a viral protein.

An Alphabody, polypeptide or composition is said to show 'cross-reactivity' for two different target proteins of interest if it is specific for (as defined herein) both of these different target proteins of interest.

The term 'monovalent' as used herein, refers to the fact that the Alphabody contains one binding site directed against or specifically binding to a site, determinant, part, domain or stretch of amino acid residues of the target of interest.

In cases where two or more binding sites of an Alphabody are directed against or specifically bind to the same site, determinant, part, domain or stretch of amino acid residues of the target of interest, the Alphabody is said to be 'bivalent' (in the case of two binding sites on the Alphabody) or multivalent (in the case of more than two binding sites on the Alphabody), such as for example trivalent.

The term 'bi-specific' when referring to an Alphabody implies that either a) two or more of the binding sites of an Alphabody are directed against or specifically bind to the same target of interest but not to the same (i.e., to a different) site, determinant, part, domain or stretch of amino acid residues of that target, or b) two or more binding sites of an Alphabody are directed against or specifically bind to different target molecules of interest. The term 'multispecific' is used in the case that more than two binding sites are present on the Alphabody.

Accordingly, a 'bispecific Alphabody' or a 'multi-specific Alphabody' as used herein, shall have the meaning of a single-chain Alphabody of the formula (N-)HRS1-L1-HRS2-L2-HRS3(-C) comprising respectively two or at least two binding sites, wherein these two or more binding sites have a different binding specificity. Thus, an Alphabody is herein considered 'bispecific' or 'multispecific' if respectively two or more than two different binding regions exist in the same, monomeric, single-domain Alphabody.

As used herein, the term 'prevention and/or treatment' comprises preventing and/or treating a certain disease and/or disorder, preventing the onset of a certain disease and/or disorder, slowing down or reversing the progress of a certain disease and/or disorder, preventing or slowing down the onset of one or more symptoms associated with a certain disease and/or disorder, reducing and/or alleviating one or more symptoms associated with a certain disease and/or disorder, reducing the severity and/or the duration of a certain disease and/or disorder, and generally any prophylactic or therapeutic effect of the Alphabodies or polypeptides that is beneficial to the subject or patient being treated.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The present inventors have identified methods of making target-binding Alphabodies, i.e., Alphabodies binding specifically to or being directed against a target protein of interest.

In addition, it has been found that target-binding Alphabodies can bind to the target with affinities at least comparable to traditional binding agents. Moreover, target-binding Alphabodies maintain the advantages identified for Alphabody scaffolds, such as the Alphabody scaffolds provided in WO 2010/066740 and EP 2 161 278. Alphabodies not only have a unique structure but also have several advantages over the traditional (immunoglobulin and non-immunoglobulin) scaffolds known in the art. These advantages include, but are not limited to, the fact that they are compact and small in size (between 10 and 14 kDa, which is 10 times smaller than an antibody), they are extremely thermostable (i.e., they generally have a melting temperature of more than 100 °C), they can be made resistant to different proteases, they are highly engineerable (in the sense that multiple substitutions will generally not obliterate their correct and stable folding), and have a structure which is based on natural motifs which have been redesigned via protein engineering techniques.

The target-binding Alphabodies according to the present invention are amino acid sequences, polypeptides or proteins having the general formula HRS1-L1-HRS2-L2-HRS3, optionally comprising additional N- and C-terminal linked groups, residues or moieties resulting in the formula N-HRS1-L1-HRS2-L2-HRS3-C. The optional N and C extensions can be, for example, a tag for detection or purification (e.g., a His-tag) or another protein or protein domain, in which case the full construct is denoted a fusion protein. For the sake of clarity, the optional extensions N and C are herein considered not to form part of a single-chain Alphabody structure, which is defined by the general formula 'HRS1-L1-HRS2-L2-HRS3'. General reference is made herein to Alphabody polypeptides which can consist of an Alphabody or comprise one or more Alphabodies, optionally having groups, residues or moieties linked thereto.

As indicated above, a heptad repeat of an Alphabody is generally represented as 'abcdefg' or 'defgabc', wherein the symbols 'a' to 'g' denote conventional heptad positions.

The 'a-positions' and 'd-positions' in each heptad unit of an Alphabody are amino acid residue positions of the coiled coil structure where the solvent-shielded (i.e., buried) core residues are located. The 'e-positions' and 'g-positions' in each heptad unit of an Alphabody are amino acid residue positions of the coiled coil structure where the amino acid residues which are partially solvent-exposed are located. In a triple-stranded coiled coil, these 'e-positions' and 'g-positions' are located in the groove formed between two spatially adjacent alpha-helices, and the corresponding amino acid residues are commonly denoted the 'groove residues'. The 'b-positions', 'c-positions' and 'f-positions' in each heptad unit of an Alphabody are the most solvent-exposed positions in a coiled coil structure.

It is noted that in the prior art, a heptad may be referred to as 'heptad repeat' because the 7-residue fragment is usually repeated a number of times in a true coiled coil amino acid sequence.

A heptad motif (as defined herein) of the type 'abcdefg' is typically represented as 'HPPHPPP', whereas a 'heptad motif of the type 'defgabc' is typically represented as 'HPPPHPP', wherein the symbol 'H' denotes an apolar or hydrophobic amino acid residue and the symbol 'P' denotes a polar or hydrophilic amino acid residue. Typical hydrophobic residues located at a- or d-positions include aliphatic (e.g., leucine, isoleucine, valine, methionine) or aromatic (e.g., phenylalanine) amino acid residues. Heptads within coiled coil sequences do not always comply with the ideal pattern of hydrophobic and polar residues, as polar residues are occasionally located at 'H' positions and hydrophobic residues at 'P' positions. Thus, the patterns 'HPPHPPP' and 'HPPPHPP' are to be considered as ideal patterns or characteristic reference motifs. Occasionally, the characteristic heptad motif is represented as 'HPPHCPC' or 'HxxHCxC' wherein 'H' and 'P' have the same meaning as above, 'C' denotes a charged residue (lysine, arginine, glutamic acid or aspartic acid) and 'x' denotes any (unspecified) natural amino acid residue. Since a heptad can equally well start at a d-position, the latter motifs can also be written as 'HCPCHPP' or 'HCxCHxx'. It is noted that single-chain Alphabodies are intrinsically so stable that they do not require the aid of ionic interactions between charged ('C') residues at heptad e- and g-positions.

A heptad repeat sequence (HRS) (as defined herein) is typically represented by (abcdefg)ₙ or (defgabc)ₙ in notations referring to conventional heptad positions, or by (HPPHPPP)ₙ or (HPPPHPP)ₙ in notations referring to the heptad motifs, with the proviso that not all amino acid residues in a HRS should strictly follow the ideal pattern of hydrophobic and polar residues. In order to identify heptad repeat sequences, and/or their boundaries, including heptad repeat sequences comprising amino acids or amino acid sequences that deviate from the consensus motif, and if only amino acid sequence information is at hand, then the COILS method of Lupas et al. (Science 252:1162-1164 (1991)) is a suitable method for the determination or prediction of heptad repeat sequences and their boundaries, as well as for the assignment of heptad positions. Furthermore, the heptad repeat sequences can be resolved based on knowledge at a higher level than the primary structure (i.e., the amino acid sequence). Indeed, heptad repeat sequences can be identified and delineated on the basis of secondary structural information (i.e., alpha-helicity) or on the basis of tertiary structural (i.e., protein folding) information. A typical characteristic of a putative HRS is an alpha-helical structure. Another (strong) criterion is the implication of a sequence or fragment in a coiled coil structure. Any sequence or fragment that is known to form a regular coiled coil structure, i.e., without stutters or stammers as described in Brown et al. Proteins 26:134-145 (1996), is herein considered a HRS. Also and more particularly, the identification of HRS fragments can be based on high-resolution 3-D structural information (X-ray or NMR structures). Finally, but not limited hereto, and unless clear evidence of the contrary exists, or unless otherwise mentioned, the boundaries to any HRS fragment may be defined as the first (respectively last) a- or d-position at which a standard hydrophobic amino acid residue (selected from the group valine, isoleucine, leucine, methionine, phenylalanine, tyrosine or tryptophan) is located.

The linkers within a single-chain structure of the Alphabodies (as defined herein) interconnect the HRS sequences, and more particularly the first to the second HRS, and the second to the third HRS in an Alphabody. Connections between HRS fragments via disulfide bridges or chemical cross-linking or, in general, through any means of inter-chain linkage, are explicitly excluded from the definition of a linker fragment (at least, in the context of an Alphabody) because such would be in contradiction with the definition of a single-chain Alphabody. A linker fragment in an Alphabody is preferably flexible in conformation to ensure relaxed (unhindered) association of the three heptad repeat sequences as an alpha-helical coiled coil structure. Further in the context of an Alphabody, 'L1' shall denote the linker fragment one, i.e., the linker between HRS1 and HRS2, whereas 'L2' shall denote the linker fragment two, i.e., the linker between HRS2 and HRS3.

The 'coiled coil' structure of an Alphabody can be considered as being an assembly of alpha-helical heptad repeat sequences wherein the alpha-helical heptad repeat sequences are as defined supra; furthermore,
- the said alpha-helical heptad repeat sequences are wound (wrapped around each other) with a left-handed supertwist (supercoiling);
- the core residues at a- and d-positions form the core of the assembly, wherein they pack against each other in a knobs-into-holes manner as defined in the Socket algorithm (Walshaw and Woolfson, J. Mol. Biol. 307:1427-1450 (2001) and reiterated in Lupas and Gruber, Adv. Protein Chem. 70:37-78 (2005));
- the core residues are packed in regular core packing layers, where the layers are defined as in Schneider et al. (Schneider et al., Fold. Des. 3:R29-R40 (1998)).

The coiled coil structure of the Alphabodies is not to be confused with ordinary three-helix bundles. Criteria to distinguish between a true coiled coil and non-coiled coil helical bundles are provided in Desmet et al. WO 2010/066740 A1 and Schneider et al. (Schneider et al., Fold. Des. 3:R29-R40 (1998)); such criteria essentially relate to the presence or absence of structural symmetry in the packing of core residues for coiled coils and helix bundles, respectively. Also the presence or absence of left-handed supercoiling for coiled coils and helix bundles, respectively, provides a useful criterion to distinguish between both types of folding.

While aforegoing criteria in principle apply to 2-stranded, 3-stranded, 4-stranded and even more-stranded coiled coils, the Alphabodies are restricted to 3-stranded coiled coils. The coiled coil region in an Alphabody can be organized with all alpha-helices in parallel orientation (corresponding to a 'parallel Alphabody' as described in EP2161278 by Applicant Complix NV) or with one of the three alpha-helices being antiparallel to the two other (corresponding to an 'antiparallel Alphabody' as described in WO 2010/066740 by Applicant Complix NV).

The alpha-helical part of an Alphabody (as defined herein) will usually grossly coincide with the heptad repeat sequences although differences can exist near the boundaries. For example, a sequence fragment with a clear heptad motif can be non-helical due to the presence of one or more helix-distorting residues (e.g., glycine or proline). Reversely, part of a linker fragment can be alpha-helical despite the fact that it is located outside a heptad repeat region. Further, any part of one or more alpha-helical heptad repeat sequences is also considered an alpha-helical part of a single-chain Alphabody.

The solvent-oriented region of (the alpha-helices of) an Alphabody (as defined herein) is an important Alphabody region. In view of the configuration of the alpha-helices in an Alphabody, wherein the residues at heptad a- and d-positions form the core, the solvent-oriented region is largely formed by b-, c- and f-residues. There are three such regions per single-chain Alphabody, i.e., one in each alpha-helix. Any part of such solvent-oriented region is also considered a solvent-oriented region. For example, a subregion composed of the b-, c- and f-residues from three consecutive heptads in an Alphabody alpha-helix will also form a solvent-oriented surface region.

Residues implicated in the formation of (the surface of) a groove between two adjacent alpha-helices in an Alphabody are generally located at heptad e- and g-positions, but some of the more exposed b- and c-positions as well as some of the largely buried core a- and d-positions may also contribute a the binding site located in a groove surface; such will essentially depend on the size of the amino acid side chains placed at these positions. Where the groove is formed by spatially adjacent alpha-helices running parallel, then the groove is formed by b- and e-residues from a first helix and by c- and g-residues of a second helix. If the said spatially adjacent alpha-helices are antiparallel, then there exist two possibilities. In a first possibility, both halves of the groove are formed by b- and e-residues (i.e., by b- and e- residues from both the first and second helix). In the second possibility, both halves of the groove are formed by c- and g-residues (i.e., by c- and g-residues from the first and second helix). The three types of possible grooves are herein denoted by their primary groove-forming (e- and g-) residues: if the helices are parallel, then the groove is referred to as an 'e/g-groove'; if the helices are antiparallel, then the groove is referred to as either an 'e/e-groove' or a 'g/g-groove'. Parallel Alphabodies (i.e., wherein all three helixes run in parallel) have three e/g-grooves, whereas antiparallel Alphabodies (i.e., comprising one antiparallel and two parallel helixes) have one e/g-groove, one e/e-groove and one g/g-groove. Any part of an Alphabody groove is also referred to herein as a groove region.

As a main object, the present invention provides methods for providing Alphabodies that specifically bind to a viral protein of interest and Alphabodies having detectable binding affinity for, or detectable in vitro activity on, a viral protein of interest, which are obtainable by the methods according to the invention. The application discloses polypeptides and compositions comprising the target protein-binding Alphabodies of the invention and the use thereof for prophylactic, therapeutic or diagnostic purposes or as research tools.

In particular embodiments, the Alphabodies, polypeptides and (pharmaceutical) compositions disclosed herein bind to a viral protein

In particular embodiments, the viral protein is a viral attachment protein and/or a viral fusion protein. Viral attachment proteins and viral fusion proteins are known to the person skilled in the art.

The viral attachment proteins envisaged in the context of the present invention include but are not limited to the gp120 protein of HIV-1 virus, the HA1 protein of influenza virus, the NA protein of influenza virus, the G protein of respiratory syncytial virus, fibre proteins in adenoviruses, the sigma 1 protein in reoviruses. The envisaged viral fusion proteins include the F protein of respiratory syncytial virus, the HA protein of Influenza A virus, the HEF protein of influenza C virus, the F protein of Simian parainfluenza virus, the F protein of Human parainfluenza virus, the F protein of Newcastle disease virus, the F2 protein of measles, the F2 protein of Sendai virus, the gp2 protein of Ebola virus, the TM protein of Moloney murine leukemia virus, the gp41 protein of HIV-1, the gp41 protein of Simian immunodeficiency virus, the gp21 protein of Human T-cell leukemia virus 1, the TM protein of Human syncytin-2, the TM protein of Visna virus, the S2 protein of Mouse hepatitis virus, the E2 protein of SARS corona virus, the E protein of Tick-borne encephalitis virus, the E2 protein of Dengue 2 and 3 virus, the E protein of Yellow Fever virus, the E protein of West Nile virus, the E1 protein of Semliki forest virus, the E1 protein of Sindbis virus, the G protein of Rabies virus, the G protein of Vesicular stomatitis virus and the gB protein of Herpes simplex virus.

In particular embodiments, the envisaged viral protein is not the gp41 protein of HIV-1.

In certain particular embodiments, the viral protein may be a viral protein which is not a viral fusion protein, such as but not limited to, a DNA polymerase, a viral T7 RNA polymerase, a viral RNA polymerase I, a viral RNA polymerase II, a viral RNA polymerase III.

The methods of the present invention are typically aimed at generating Alphabodies, polypeptides and compositions which can bind to one or more of the above-listed or - mentioned viral proteins.

The specific conformation and/or secondary and/or tertiary and/or quaternary structure of the viral fusion protein is not considered critical in the context of the present invention. Thus, the viral fusion protein of interest may be characterized by any conformation and/or secondary and/or tertiary and/or quaternary structure.

In particular embodiments, the Alphabodies disclosed herein may bind to the viral fusion protein, which is in its native, usually oligomeric form. Also, the Alphabodies may bind to the viral fusion protein of interest in isolated, soluble form. Further, the Alphabodies may bind to the viral fusion protein of interest as precursor or as mature protein. Further, the Alphabodies may bind the viral fusion protein of interest in its native conformational state, in a mechanistic intermediate state (for example, in a fusion-activated or prefusion intermediate state), or in a postfusion state. Where the viral protein is a viral fusion protein, the Alphabodies disclosed herein may typically bind their epitopes of the viral proteins after they have become accessible as a result of triggering by cellular receptors or by a pH drop. However, the Alphabodies may also or alternatively bind the viral fusion protein of interest in a free, unliganded state, or in a receptor-or ligand-bound state. In further particular embodiments, the Alphabodies may bind the Alphabodies of the invention may bind to a domain of a viral fusion protein, which is responsible for attachment to a target cell (or to a target cell-displayed receptor), or to a domain responsible for membrane fusion. Further, the Alphabodies may bind to a fragment of a full viral fusion protein, such as, but not limited to a domain or a subregion. Such fragment may be obtained, for example, by enzymatic proteolysis or by reduction of disulfide bonds. In particular embodiments, the viral fusion protein is not HIV-1 envelope glycoprotein (HIV-1 Env). In further particular embodiments, the target protein is not HIV-1 gp41. However, according to particular embodiments, the target protein is a viral protein provided it is not any of subregions HR1 or HR2 of HIV-1 gp41.

In particular embodiments, the Alphabodies, and/or polypeptides disclosed herein bind to the viral protein of interest with a dissociation constant (KD) of less than about 1 micromolar (1 µM), and preferably less than about 1 nanomolar (1 nM) [i.e., with an association constant (KA) of about 1,000,000 per molar (10⁶ M⁻¹, 1E6 /M) or more, and preferably about 1,000,000,000 per molar (10⁹ M⁻¹, 1E9 /M) or more]. In particular embodiments, an Alphabody, polypeptide or composition will bind to the target protein of interest with a kOff ranging between 0.1 and 0.0001 s⁻¹ and/or a kOn ranging between 1,000 and 1,000,000 M⁻¹ s⁻¹.

The ability of the Alphabodies and/or polypeptides provided herein to bind to a viral protein with a particular affinity make them suitable for use in a number of applications, including screening, detection, diagnostic and therapeutic applications as will be described more in detail herein below.

In particular embodiments, the Alphabodies produced by the methods of the present invention are capable of inhibiting, reducing and/or preventing the interaction between a target protein of interest, in particular a viral protein of interest or a subdomain or subfragment thereof, and its natural binding partner, or, inhibiting, reducing and/or preventing the activity of a viral target protein of interest, or, inhibiting, reducing and/or preventing one or more biological or physiological mechanisms, effects, responses, functions pathways or activities in which the viral target protein of interest is involved, such as by at least 10%, but preferably at least 20%, for example by at least 50%, at least 60%, at least 70%, at least 80%, at least 90%, at least 95% or more, as measured using a suitable in vitro, cellular or in vivo assay, compared to the activity of the viral target protein of interest in the same assay under the same conditions but without using the Alphabody, polypeptide or composition.

Thus, in particular embodiments, the Alphabodies, polypeptides and compositions obtaind by the methods of the invention can reduce or inhibit binding of that viral protein to its receptor or can reduce, inhibit or prevent the interaction between different domains, regions, subregions or parts of that viral protein compared to the binding of the viral protein to its receptor or the interaction between different domains, regions, subregions or parts of that viral protein in the absence of such molecules, and this by at least 10%, but preferably at least 20%, for example by at least 50%, at least 60%, at least 70%, at least 80%, at least 90%, at least 95% or more, as determined by a suitable assay known in the art. Alternatively, the binding of the Alphabodies to a viral protein is such that it still allows the viral protein to bind to its receptor, but prevents, reduces or inhibits viral membrane fusion or viral entry into a target cell or viral infection by at least 10%, but preferably at least 20%, for example by at least 50%, at least 60%, at least 70%, at least 80%, at least 90%, at least 95% or more, as determined by a suitable assay known in the art.

Accordingly, in particular embodiments the Alphabodies, polypeptides and compositions disclosed herein can be used for the prevention and treatment of viral diseases which are characterized by viral-mediated biological pathway(s) in which a viral protein, such as a viral protein of interest, or a subdomain or subfragment thereof, is involved.

The present invention provides methods for obtaining the target-specific Alphabodies according to the invention, and more particularly for obtaining Alphabodies specifically binding to or directed against viral proteins. These methods are based on the concept of random library screening.

The target-specific Alphabodies according to the present invention can be obtained by methods which involve generating a random library of Alphabody polypeptides and screening this library for an Alphabody capable of specifically binding to a target of interest, and in particular to a viral protein of interest.

A first step in the methods according to the invention comprises the production of a library (i.e., collection or set) of Alphabody polypeptide sequences, each of which differ from each other in at least one of a defined set of 5 to 20 variegated amino acid residue positions. Therefore, the sequences within a library of Alphabody polypeptides differ from each other at particular amino acid positions that are comprised in a selected or defined set. Accordingly, the term 'different-sequence' refers to the occurrence of sequence variation or sequence differences within a defined set of amino acid residue positions between two or more Alphabody polypeptides of the libraries.

A library or collection of Alphabody sequences may contain any suitable number of different Alphabody sequences, such as at least 2, at least 5, at least 10, at least 50, at least 100, at least 1000, at least 10,000, at least 10⁵, at least 10⁶, at least 10⁷, at least 10⁸, at least 10⁹ or more different-sequence (single-chain) Alphabodies.

More particularly, a library or collection of different Alphabody sequences according to the present invention contains at least 100 different-sequence Alphabody polypeptides, such as at least 200, at least 300, at least 400, at least 500, such as at least 1000, at least 10000, at least 10⁵, at least 10⁶, at least 10⁷, at least 10⁸, at least 10⁹ or more sequences.

In particular embodiments, a set, collection or library (as used interchangeably herein) of Alphabody sequences contains at least 100 different-sequence Alphabody polypeptides.

In addition, the single-chain Alphabody libraries disclosed herein are characterized in that the different-sequence single-chain Alphabody polypeptides comprised in those libraries differ from each other in at least one of a defined set of variegated amino acid residue positions. Accordingly, in particular embodiments, the different Alphabody sequences, also referred to herein as different-sequence (single-chain) Alphabodies, comprised in the libraries, only differ from each other in a defined, i.e. fixed or predetermined, set of amino acid residue positions. Such a defined set of variegated amino acid residue positions consists of a number of particular amino acid residue positions, which are characterized by variety or diversity of amino acid residue types when the different-sequence (single-chain) Alphabodies within the produced library are compared to each other.

The notion 'variegated amino acid residue position', when referring to a library of different-sequence Alphabodies, refers to an amino acid residue position at which at least two different amino acid residue types (amino acid residues of a defined type, for example natural amino acid residue types) are located when at least two of the amino acid sequences of the different-sequence Alphabodies from the said library of Alphabodies are compared to each other (note that these positions will not differ for any two different-sequence Alphabodies of the library, but that the library comprises at least two different-sequence Alphabodies which differ in this amino acid residue position). A 'set of variegated amino acid residue positions', when referring to a library of different-sequence Alphabodies, refers to the set of amino acid residue positions at which at least two different amino acid residue types are located when at least two of the amino acid sequences of the different-sequence Alphabodies from the said library of Alphabodies are compared to each other (note that these positions will not differ for any two different-sequence Alphabodies of the library). A 'defined set of variegated amino acid residue positions', when referring to a library of different-sequence Alphabodies, refers to the specific set of amino acid residue positions at which at least two different amino acid residue types are located when all amino acid sequences from the said library of different-sequence Alphabodies are simultaneously compared to each other. Thus, the simultaneous comparison of all amino acid sequences from the said library of different-sequence Alphabodies, and the identification of amino acid residue positions at which at least two different amino acid residue types are located in such simultaneous comparison, allows to identify the said defined set of variegated amino acid residue positions in an Alphabody library. It is herein submitted that a skilled person will known how to determine the sequences in a library of sequences such as a single-chain Alphabody library. It is herein further understood that Alphabody sequences can be compared both at the level of amino acid sequences or nucleotide sequences representing (encoding) these amino acid sequences.

A preferred method to compare two or more different-sequence Alphabodies is based on a pair-wise or multiple sequence alignment, generated by a known computer algorithm for automated sequence alignment such as NCBI Blast. Alternatively, two or more different-sequence Alphabodies can be compared on the basis of a pair-wise or multiple sequence alignment which is generated by a skilled user, such method of alignment also being known as manual sequence alignment. Both of the techniques of automated and manual sequence alignment applied to different-sequence Alphabodies can be based on the maximization of global sequence identity or global sequence similarity (or homology or correspondence), or on the maximization of sequence identity or similarity of the core amino acid positions (i.e., the heptad a- and d- positions as defined herein) of the different-sequence Alphabodies.

Accordingly, a defined set of variegated amino acid residue positions can be deduced from an amino acid or nucleotide sequence comparison of all, or at least a representative subset of all different-sequence Alphabodies in an Alphabody library. It is also acknowledged that, upon generating an Alphabody library, so-called unintended mutations, insertions or deletions may occur. Such unintended mutations, insertions or deletions are nucleotide or amino acid mutations, insertions or deletions that occur at positions which were not intended to be variegated at the time of designing or generating the said Alphabody library. As will be acknowledged by a skilled person, and on condition that the said Alphabody library was generated with state-of-the-art technology, such unintended mutations, insertions or deletions will occur only sporadically and in a scattered fashion (i.e., at different positions) within the Alphabody library sequences. Preferably, such unintended mutations, insertions or deletions will occur at any given position with a frequency of less than 10%, more preferably less than 5%, more preferably less than 2%, 1%, or even less than 1%. Accordingly, unintended mutations, insertions or deletions within an Alphabody library can be identified on the basis of their low frequency as compared to the variability observed at positions showing intended sequence variation. Thus, a preferred method to determine a defined set of variegated amino acid residue positions within an Alphabody library, without possessing knowledge about the design or production of said library, is by determining the nucleotide or amino acid sequences of at least a representative subset of sequences contained within this library, followed by comparing all determined sequences in a (preferably multiple) alignment, followed by identifying the positions at which sequence variation is observed, followed by identifying the frequencies of the nucleotides or amino acid residue types observed at each variable position, followed by identifying the positions having a mutation, insertion or deletion frequency higher than a given cutoff percentage, wherein this cutoff percentage is set at 10%, 5%, 2%, 1% or even less than 1% such as 0.5%, 0.1% or 0% (in which case all observed variations are included in the defined set of variegated amino acid residue positions. Alternatively, if knowledge about the original design or production of a single-chain Alphabody library is available, then the set of variegated amino acid residue positions is this library is preferably based on said knowledge instead of on the analysis of the library itself. Methods to design single-chain Alphabody libraries, including the selection of variegated positions, are described below.

According to the methods of the present invention, the number of variegated amino acid residue positions in such a defined set can range from 5 to 20 amino acid residue positions. Thus, a defined set of variegated amino acid residue positions in a library may comprise 5 to 20, such as 5, 6, 7, 8, 9, 10, 11, 12, 13, 14, 15, 16, 17, 18, 19, or 20, defined variegated amino acid residue positions.

In the methods of the present invention, the different-sequence Alphabody polypeptides comprised in a library can differ from each other in at least one amino acid residue position of such a defined set of 5 to 20 positions. Thus, for example when the defined set of variegated amino acid residue positions in a library comprises a set of 13 variegated amino acid residue positions, the different-sequence Alphabody polypeptides in the libraries can differ from each other in 1, 2, 3, 4, 5, 6, 7, 8, 9, 10, 11, 12, or all 13 of these amino acid residue positions. Accordingly, it is clear that the different-sequence Alphabody polypeptide sequences comprised in a library can be distinguished from each other by the sequence difference(s) present in the defined set of 5 to 20 variegated amino acid residue positions, with the proviso that additional, preferably sporadic, unintended mutations, insertions or deletions may occur at positions other than those of the defined set of variegated positions.

The Alphabodies obtainable by the methods of the present invention form a single-chain, 3-stranded alpha-helical coiled coil structure which is represented as HRS1-L1-HRS2-L2-HRS3. The heptad repeat sequences (HRS) 1, 2 and 3 each adopt an alpha-helical conformation and are referred to as (alpha-)helices of the Alphabody. These three helices (being present in an Alphabody) are typically referred to as helices A, B and C, respectively.

Making use of the Alphabody single-chain coiled coil scaffold, different types of Alphabody libraries can be generated, depending on where the set of variegated amino acid residue positions is located within the Alphabody structure. The location of the set of variegated amino acids determines the type and the location of the binding site that is generated on the Alphabodies by application of the methods of the invention. Reference can be made in this context to 'groove libraries' (wherein binding sites are predominantly formed by amino acid residues located in the groove between two of the three helices of the Alphabody), 'helix libraries' (wherein binding sites are predominantly formed by amino acid residues located at the solvent-oriented side of one of the three alpha-helices of the Alphabody), or 'loop libraries' (wherein binding sites are predominantly formed by amino acid residues located in one or more of the Alphabody linker sequences fulfilling the role of a loop).

Libraries wherein the variegated amino acid residue positions are located exclusively (i.e., for 100%) in either a groove, surface or linker of the Alphabody are referred to herein as 'pure groove', 'pure surface', or 'pure loop' libraries. However, as will be detailed herein below, the methods envisage the use of Alphabody libraries, which need not be 'pure' groove, surface or loop libraries.

In one embodiment of the methods invention, Alphabody libraries are used wherein the variegated amino acid residue positions are located predominantly in the groove between two of the three helices of the Alphabody. In these embodiments, in the Alphabodies obtained by the methods of the invention, the binding site for binding to a viral protein is formed predominantly by amino acid residue positions located in the groove between two of the three alpha-helices of the Alphabody.

Residues implicated in the formation of (the surface of) a groove between two adjacent alpha-helices in an Alphabody are generally located at the heptad e- and g-positions, but some of the more exposed b- and c-positions as well as some of the largely buried core a- and d-positions may also contribute to a groove surface; such will essentially depend on the size of the amino acid side chains placed at these positions.

Depending on the nature of the Alphabodies generated, being folded either as parallel or antiparallel single-chain coiled coils, the positions within the Alphabody sequence which need to be variegated in order to obtain a groove library will vary.

When the two spatially adjacent alpha-helices of the Alphabody (i.e., those between which the groove for binding is located) run parallel, as is the case for all pairs of helices in parallel Alphabodies and for helices A and C in antiparallel Alphabodies, then a binding site located in the groove can be formed by variegation of at least some of the e-residues from a first helix and at least some of the g-residues from a parallel second helix. In addition to these residues, the binding site may optionally further be formed by variegating residues at b-positions in the first helix and/or residues at c-positions in the parallel second helix. Thus, in these embodiments, in order to obtain Alphabodies directed against a viral protein of interest and wherein the binding site is located in a e/g-groove, the variegated amino acid residue positions of the Alphabody libraries used in the generation of target-specific Alphabodies are located at heptad e-positions in a first alpha-helix of the Alphabody polypeptides and at heptad g-positions in a second alpha-helix, parallel to the first alpha-helix, and optionally also at heptad b-positions in the first alpha-helix and/or at heptad c-positions in the second alpha-helix of the Alphabody polypeptides. In particular embodiments of the invention, it is envisaged that Alphabody libraries are used wherein the variegated amino acid residue positions are predominantly (i.e., for at least 70%) located at heptad e- (and optionally also b-) positions in a first alpha-helix of the Alphabody polypeptides and at heptad g- (and optionally also c-) positions in a second alpha-helix, parallel to the first alpha-helix.

Alternatively or additionally, use can be made of Alphabody scaffolds comprising two spatially adjacent alpha-helices that are positioned antiparallel (if a binding site located in the groove between these antiparallel helices is envisaged). This is typically the case for helices A and B or B and C in antiparallel Alphabodies. In these embodiments, there are typically two possibilities for the type of variegated amino acid residue positions that need to be variegated to ensure that the binding site is formed by the groove between antiparallel helices.

In a first possibility, the groove between two antiparallel helices is formed by at least some of the e-residues from a first helix and at least some of the e-residues from an antiparallel second helix. Thus, in order to obtain a binding site formed within such e/e-groove, at least these amino acid residue positions are variegated. In addition to these residues, such a binding site may optionally further be formed by residues at b-positions in the first helix and/or residues at b-positions in the antiparallel second helix. Thus, in these embodiments, in order to obtain Alphabodies directed against a viral protein of interest and wherein the binding site is located in a e/e-groove, the variegated amino acid residue positions are located at heptad e-positions in a first alpha-helix of the Alphabody polypeptides and at heptad e-positions in a second alpha-helix, antiparallel to the first alpha-helix, and optionally also at heptad b-positions in the first alpha-helix and/or at heptad b-positions in the second alpha-helix of the Alphabody polypeptides. In particular embodiments of the of the invention, it is envisaged that Alphabody libraries are used wherein the variegated amino acid residue positions are predominantly (i.e., for at least 70%) located at heptad e- (and optionally also b-) positions in a first alpha-helix of the Alphabody polypeptides and at heptad e- (and optionally also b-) positions in a second alpha-helix, antiparallel to the first alpha-helix.

In a second possibility, the groove between two antiparallel helices is formed by at least some of the g-residues from a first helix and at least some of the g-residues from an antiparallel second helix. Thus, in order to obtain a binding site formed within such g/g-groove, at least these amino acid residue positions are variegated. In addition to these residues, such a binding site may optionally further be formed by residues at c-positions in the first helix and/or residues at c-positions in the antiparallel second helix. Thus, in these embodiments, in order to obtain Alphabodies directed against a viral protein of interest and wherein the binding site is located in a g/g-groove, the variegated amino acid residue positions are located at heptad g-positions in a first alpha-helix of the Alphabody polypeptides and at heptad g-positions in a second alpha-helix, antiparallel to the first alpha-helix, and optionally also at heptad c-positions in the first alpha-helix and/or at heptad c-positions in the second alpha-helix of the Alphabody polypeptides. In particular embodiments of the of the invention, it is envisaged that Alphabody libraries are used wherein the variegated amino acid residue positions are predominantly (i.e., for at least 70%) located at heptad g- (and optionally also c-) positions in a first alpha-helix of the Alphabody polypeptides and at heptad g- (and optionally also c-) positions in a second alpha-helix, antiparallel to the first alpha-helix.

Thus, it will be clear that parallel Alphabodies, which have three alpha-helices each oriented parallel to one another, contain three e/g-grooves. Antiparallel Alphabodies, on the other hand, which comprise two alpha-helices that are parallel to each other and one alpha-helix that runs antiparallel, contain one e/g-groove, one e/e-groove and one g/g-groove. Any part of an Alphabody groove is also considered a groove region.

In principle, 'pure' groove libraries are Alphabody libraries characterized in that the variegated amino acid residue positions in such libraries are all located at heptad e- and b-or g- and c-positions in a first alpha-helix of the Alphabody polypeptides and at heptad e-and b- or g- and c-positions in a second alpha-helix of the Alphabody polypeptides. It has been found by the present inventors that Alphabody libraries which are characterized in that the variegated amino acid residue positions in such libraries are not exclusively but only predominantly located at heptad e- and b- or g- and c-positions in a first alpha-helix of the Alphabody polypeptides and at heptad at heptad e- and b- or g- and c-positions in a second alpha-helix of the Alphabody polypeptides, generate more and better target-specific Alphabodies. Accordingly, the methods of the present invention specifically envisage the use of Alphabody libraries wherein the variegated amino acid residue positions in such libraries, are predominantly (i.e., for at least 70%), but not exclusively located at heptad e-and b- or g- and c-positions in a first alpha-helix of the Alphabody polypeptides and at heptad e- and b- or g- and c-positions in a second alpha-helix of the Alphabody polypeptides. In particular embodiments, the variegated amino acid residue positions in the Alphabody libraries used are located for at least 70% at the indicated groove-forming positions. In further particular embodiments, at least one of the variegated amino acid residue positions in the libraries is located outside the positions corresponding to the heptad e- and b- or g- and c-positions in a first alpha-helix of the Alphabody polypeptides and corresponding to the heptad e- and b- or g- and c-positions in a second alpha-helix of the Alphabody polypeptides.

Additionally or alternatively, it is envisaged that the methods of the present invention comprise the use of Alphabody libraries in which the binding site is formed predominantly by the surface of one of the three helices of the Alphabody. Accordingly, in these embodiments, Alphabody libraries are used in which the variegated amino acid residue positions are located predominantly at the solvent-oriented side of one of the three helices of the Alphabody polypeptides. Such Alphabody libraries are referred to herein as 'helix surface libraries' or 'helix libraries'.

The amino acid residue positions considered to be at the surface of the Alphabody can be located in either helix A, B or C. In particular embodiments, the Alphabody library contains Alphabodies in which the variegated amino acid residues correspond to the fully solvent-exposed amino acid residues of helix C. The most protruding and thus solvent-oriented residues are located at b-, c- and f-positions in each of the alpha-helices of an Alphabody. Thus, in particular embodiments, the Alphabody libraries comprise variegated amino acid residue positions that are located at heptad b-, c- and f-positions in one alpha-helix of the Alphabody polypeptides.

In principle, 'pure' helix surface libraries are Alphabody libraries characterized in that the variegated amino acid residue positions in such libraries are all located at heptad b-, c-and f-positions in one alpha-helix of the Alphabody polypeptides. It has been found by the present inventors that Alphabody libraries which are characterized in that the variegated amino acid residue positions are not exclusively but only predominantly located at heptad b-, c- and f-positions of an alpha-helix in the Alphabody polypeptides generate more and better target-specific Alphabodies. Accordingly, the methods of the present invention specifically envisage the use of Alphabody libraries wherein the variegated amino acid residue positions in such libraries are predominantly (i.e., for at least 70%), but not exclusively located at heptad b-, c- and f-positions of an alpha-helix of the Alphabody polypeptides. In particular embodiments, the variegated amino acid residue positions in the Alphabody libraries used are located for at least 70% at the indicated solvent-exposed positions. In further particular embodiments, at least one of the variegated amino acid residue positions in the libraries is located outside the positions corresponding to the heptad b-, c- and f-positions of an alpha-helix of the Alphabody polypeptides.

Additionally or alternatively, it is envisaged that the methods of the present invention comprise the use of Alphabody libraries in which the binding site is formed predominantly by amino acid residue positions located in one of the Alphabody linker sequences interconnecting the alpha-helices. In a parallel Alphabody, the two linkers interconnect distal ends of the coiled coil structure (i.e., they form a link between a helix C-terminus and a helix N-terminus located at the opposite end). In an antiparallel Alphabody, the two linkers interconnect proximal ends of the coiled coil structure (i.e., they form a link between a helix C-terminus and a helix N-terminus located at the same end). In both types of Alphabodies, the linkers are deemed to be conformationally flexible. In addition, the linkers in both types of Alphabodies are also deemed to be flexible with respect to their amino acid sequence. Thus, the linkers in both types of Alphabodies may be subjected to amino acid sequence variation without significant effects on the folding and stability of the Alphabody coiled coil scaffold. Consequently, Alphabody 'loop' or 'linker libraries' are provided wherein sequence variegation is introduced within the amino acid positions of any of the linker fragments in any of the parallel or antiparallel Alphabody types. In particular embodiments, libraries are generated or used wherein all of the amino acid residue positions of one linker fragment are variegated. In further particular embodiments, a selection of the residue positions located near the middle of a linker fragment are variegated (in order not to interfere with the alpha-helical termini). In further particular embodiments, a selection of residue positions near one of the ends of a linker fragment may be varied; although this increases the risk of interference with the alpha-helical terminus, the Alphabodies will be generally stable enough to tolerate such potential interference. In still further particular embodiments, alternating residue positions over the partial or complete length of the loop sequence may be variegated.

'Pure' linker or loop libraries are Alphabody libraries characterized in that the variegated amino acid residue positions in such libraries, are all located within a linker fragment of the Alphabody polypeptides. It has been found by the present inventors that Alphabody libraries which are characterized in that the variegated amino acid residue positions are not exclusively but only predominantly located at linker positions in the Alphabody polypeptides generate more and better target-specific Alphabodies. Accordingly, the methods of the present invention specifically envisage the use of Alphabody libraries wherein the variegated amino acid residue positions in such libraries are predominantly (i.e., for at least 70%), but not exclusively located in one of the linkers of the Alphabody polypeptides. In particular embodiments, the variegated amino acid residue positions in the Alphabody libraries used are located for at least 70% at positions within one of the Alphabody linker fragments. In further particular embodiments, at least one of the variegated amino acid residue positions in the libraries is located outside the amino acid residue positions of a linker fragment of the Alphabody polypeptides.

As indicated above, in particular embodiments of the methods of the invention, Alphabody libraries are used which are characterized in that the set of variegated amino acids contains at least one, more particularly two or more amino acid residue positions which are located outside, respectively, a groove, helix surface or linker fragment of an Alphabody such that the binding site is predominantly formed, respectively, by that groove, helix surface or linker fragment. In these embodiments, the percentage of variegated amino acid positions within the groove, helix surface or linker fragment of an Alphabody having a binding site that is predominantly formed by that groove, helix surface or linker fragment, respectively, is less than 100%. However, the percentage of variegated amino acid positions that is located within the groove, helix surface or linker fragment is typically at least 70%.

Thus, in particular embodiments of the present invention, the libraries used are not pure groove, pure surface or pure loop libraries. More particularly, at least 70% but not all of the variegated amino acid positions, such as for example less than 95%, such as less than 90%, or less than 85% of the variegated amino acid positions are located within either a groove, a helix surface or a linker fragment of the Alphabody.

Depending on the number of amino acid residue positions variegated, the percentages described above will correspond to a different number of actual amino acid residue positions. Accordingly, as will be clear from the above, in particular embodiments of the methods of the invention, Alphabody libraries are used in which for example at least 5% (i.e., at least 1 position of the 5 to 20 variegated positions), or particularly at least 10% (i.e., at least 1 or at least 2 positions), or particularly at least 15% (i.e., at least 1 to at least 3 positions), or particularly at least 20% (i.e., at least 1 to at least 4 positions), or particularly at least 25% (i.e., at least 2 to at least 5 positions), or particularly 30% (i.e., between 2 and 6 positions) of these 5 to 20 positions are located at positions other than:
(i) at heptad e- or g-positions in a first alpha-helix of the Alphabody polypeptides and at heptad e- or g-positions in a second alpha-helix, and optionally at heptad b- or c-positions in the first alpha-helix of the Alphabody polypeptides and/or at heptad b- or c-positions in the second alpha-helix of the Alphabody polypeptides, such as
   (i1) at heptad e-positions in a first alpha-helix of the Alphabody polypeptides and at heptad g-positions in a second alpha-helix, parallel to the first alpha-helix, and optionally at heptad b-positions in the first alpha-helix of the Alphabody polypeptides and/or at heptad c-positions in the second alpha-helix of the Alphabody polypeptides,
      or
   (i2) at heptad e-positions in a first alpha-helix of the Alphabody polypeptides and at heptad e-positions in a second alpha-helix, antiparallel to the first alpha-helix, and optionally at heptad b-positions in the first alpha-helix of the Alphabody polypeptides and/or at heptad b-positions in the second alpha-helix of the Alphabody polypeptides,
      or
   (i3) at heptad g-positions in a first alpha-helix of the Alphabody polypeptides and at heptad g-positions in a second alpha-helix, antiparallel to the first alpha-helix, and optionally at heptad c-positions in the first alpha-helix of the Alphabody polypeptides and/or at heptad c-positions in the second alpha-helix of the Alphabody polypeptides,
      or
(ii) at heptad b-, c- and f-positions in one alpha-helix of the Alphabody polypeptides,
   or
(iii) at positions in a linker fragment connecting two consecutive alpha-helices of the Alphabody polypeptides.

Accordingly, the different-sequence (single-chain) Alphabodies in a library differ in a defined set of 5 to 20 amino acid residue positions, wherein, for each library, at least 70% (i.e., at least 4 to at least 14 positions) of these 5 to 20 positions, such as at least 75% (i.e., at least 4 to at least 15 positions), at least 80% (i.e., at least 4 to at least 16 positions), at least 85% (i.e., at least 4 to at least 17 positions), such as at least 90% (i.e., at least 5 to at least 18 positions), for example at least 95% (i.e., at least 5 to at least 19 positions), or more, such as 100% (i.e., all 5 to 20 positions) are located either
(i) at a groove formed by or between two adjacent alpha-helices, or
(ii) at a solvent-oriented surface of one alpha-helix, or
(iii) within one of the linker fragments interconnecting two alpha-helices,
of each the Alphabody polypeptides in the library; more particularly these positions are located at the positions recited for each of the options above.

It is noted that for the Alphabody libraries wherein the variegated amino acid residue positions are located primarily (i.e., at least 70%) in the groove, the remaining variegated positions that are located elsewhere than at the said heptad e-, g-, b- or c-positions may for example be located at heptad a-positions, at heptad d-positions, at heptad f-positions or at amino acid residue positions in the linkers of the Alphabody. The a-positions and d-positions (also referred to as core residues) in each heptad repeat sequence of an Alphabody are amino acid residue positions of the coiled coil structure which form essentially the solvent-shielded (i.e., buried) part of the Alphabody. It is envisaged that in most Alphabody groove libraries used in the context of the present invention, all or some of these core residues are kept conserved in order to maintain the stability of the Alphabodies. In these embodiments, the remaining variegated positions may be located for instance at linker residue positions or heptad f-positions.

Similarly for the Alphabody libraries wherein the variegated amino acid residue positions are located primarily (i.e., at least 70%) at the solvent-oriented surface of an Alphabody helix, the remaining variegated positions that are not located at the said heptad b-, c-, or f-positions may for example be located at heptad e-, g-, a- or d-positions or at amino acid residue positions in the linkers of the Alphabody. As detailed above, it is envisaged that in most Alphabody helix surface libraries used in the context of the present invention, all or some of the core residues (at a- and d-positions) are kept conserved in order to maintain the stability of the Alphabodies. In these embodiments, the remaining variegated positions may be located for instance at heptad e- or g-positions or at linker residue positions.

Finally, for the Alphabody libraries wherein the variegated amino acid residue positions are located primarily (i.e., at least 70%) within a linker fragment of the Alphabody, the remaining variegated positions that are not located within the linker fragment may for example be located at heptad e-, g-, f-, b-, c-, a- or d-positions or at amino acid residue positions in the other of the two linker fragments of the Alphabody. Again, the a-positions and d-positions in each heptad unit of an Alphabody will in some embodiments not be varied to ensure stability. In these embodiments, the remainder of the variegated amino acid residue positions may be located at heptad e-, g-, f-, b-, or c-positions or at amino acid residue positions in the other of the two linker fragments of the Alphabody.

When referring to variegated amino acid residue positions located within the groove formed by or between two adjacent alpha-helices of each of the Alphabody polypeptides, it is meant that these variegated amino acid residue positions are located within one and the same groove, i.e., a groove formed by the two same adjacent alpha-helices, in each of the Alphabody polypeptides comprised in a library. Similarly, when referring to variegated amino acid residue positions located within one alpha-helix of the Alphabody polypeptides, it is meant that these variegated amino acid residue positions are located within one and the same alpha-helix, in each of the Alphabody polypeptides comprised in a library. Also, when referring to variegated amino acid residue positions located at positions within a linker fragment interconnecting two consecutive alpha-helices, it is meant that these variegated amino acid residue positions are located within one and the same linker fragment interconnecting two consecutive alpha-helices in each of the Alphabody polypeptides comprised in a library.

However, it can be envisaged that different groove, surface or loop libraries are combined for use in the methods of the present invention. More particularly, it can be envisaged that a helix surface library comprising variegated amino acid positions primarily located on the surface of helix C is combined with a helix surface library comprising variegated amino acid positions primarily located on the surface of helix A and/or with a helix surface library comprising variegated amino acid positions primarily located on the surface of helix B. Similarly, it can be envisaged that a groove library comprising variegated amino acid positions primarily located within a e/g-groove is combined with a groove library comprising variegated amino acid positions primarily located within a e/e-groove and/or with a groove library comprising variegated amino acid positions primarily located within a g/g-groove. Similarly, it can be envisaged that a linker library comprising variegated amino acid positions primarily located within a first linker fragment is combined with a linker library comprising variegated amino acid positions primarily located within a second linker fragment. Finally, it can be envisaged that one or more helix surface libraries are combined with one or more groove libraries and/or with one or more linker libraries. A practical way of combining single-chain Alphabody libraries can be accomplished, for example, by mixing different libraries in about equal amounts.

The libraries used in the context of the present invention contain sequence variations between Alphabody polypeptides, wherein this sequence variation exclusively resides in 5 to 20 defined amino acid residue positions, of which at least 70% of these positions are located either within a groove formed between two adjacent alpha-helices, or within one alpha-helix or at positions within a linker (fragment) connecting two consecutive alpha-helices of the Alphabody polypeptides. Indeed, given the unique structural features of the Alphabody scaffold, three conceptually different types of randomization, namely within a concave groove, within a convex helix surface and within an unstructured linker between helices, can be designed.

In particular embodiments of the present invention, the constant, non-variegated part of the single-chain Alphabody polypeptides that are present in the single-chain Alphabody libraries does not correspond to a naturally occurring protein sequence, and thus the sequence representing the non-variegated part of the scaffold is of non-natural origin. Indeed, typically, the constant, non-variegated part of the Alphabody polypeptides in a library is an artificial sequence.

It will be understood by the skilled person that the Alphabody libraries comprising the variegations as described above, for use in the methods of the present invention, are typically generated by recombinant DNA techniques. More particularly, libraries of nucleic acid sequences encoding Alphabodies each differing in particular amino acid positions can be obtained by site-directed or random mutagenesis of a template sequence. As will be acknowledged by a skilled person, random amino acid residues can be introduced at specific positions in an amino acid sequence by, for example, selecting (introducing) 'NNK' or 'NNS' codons at corresponding positions in the nucleotide sequence encoding said amino acid sequence.

Thus, the generation of a (partially) randomized single-chain Alphabody library requires the (partial) randomization of specific positions within a template or standard or reference Alphabody scaffold sequence. Methods for producing such libraries are known to the skilled person and commercial services are available for generating such libraries. The nucleotide(s) determining the relevant amino acid residues at the positions of interest are mutated in different ways such as to obtain a library of nucleotide sequences encoding different Alphabodies.

A template Alphabody scaffold sequence is the sequence of a reference Alphabody which has been selected on the basis of its (near-) optimal physico-chemical properties. As demonstrated in the examples in WO 2010/066740, single-chain Alphabodies generally have a high thermal (i.e., thermodynamic) stability, a high solubility, a high resistance to variations in pH, and, importantly, a high tolerability to amino acid sequence variation. Such single-chain Alphabodies, or other single-chain Alphabodies having suitable physico-chemical properties such as a high tolerability to amino acid sequence variation, can be selected as reference or template Alphabody scaffolds (or scaffold sequences) for the generation of single-chain Alphabody libraries.

The variegation envisaged in the libraries used in the methods of the invention is envisaged to encompass both naturally occurring and synthetic amino acid residues. However, in particular embodiments of the invention, the variegated amino acid residue positions are exclusively occupied by naturally occurring amino acid types, such as for instance but not limited to natural amino acid types, such as glycine, alanine, proline, asparagine, aspartic acid, glutamine, glutamic acid, histidine, arginine, lysine, threonine, serine, cysteine, leucine, isoleucine, methionine, phenylalanine, tyrosine, tryptophan and valine.

In order to obtain the target-specific Alphabodies, the methods of the invention further comprise the step of selecting from the single-chain Alphabody library at least one single-chain Alphabody having detectable binding affinity for, or detectable in vitro activity on, the viral protein of interest.

Selection or screening methods for obtaining Alphabodies binding to a target protein of interest are known in the art and will be detailed below. It is noted that the screening of an Alphabody library may be performed in different ways, and that the screening method will be adjusted to the form in which the Alphabody library is provided. Indeed, the Alphabody libraries used in the methods of the present invention can be provided in different forms, and can be but are not limited to protein libraries, nucleic acid libraries, vector libraries or host cell libraries.

In particular embodiments, the libraries used in the context of the present invention are libraries of host cells, wherein each host cell comprises one member of a nucleic acid or vector library, each such nucleic acid or vector library member encoding a single-chain Alphabody (polypeptide). More particularly, the libraries are libraries of host cells wherein Alphabodies are expressed.

In particular embodiments, the Alphabodies of the library are displayed on the surface of a phage particle, a ribosome, a bacterium, a yeast cell, a mammalian cell or any other suitable (micro)organism, so as to facilitate screening or selection to isolate the desired Alphabody sequences having detectable binding affinity for, or detectable in vitro activity on, the viral protein of interest. Suitable methods, techniques and host organisms for displaying and selecting or screening (a set, collection or library of) variegated polypeptide sequences or nucleotide sequences encoding such variegated polypeptide sequences, and which are applicable to Alphabodies, are known to the person skilled in the art. Such methods are described, for example, in Georgiou et al., Nat. Biotechnol. 15:29-34 (1997); Wittrup, Curr. Opin. Biotechnol. 12:395-399 (2001); Lipovsek and Pluckthun, J. Immunol. Methods 290:51-67 (2004); Reiersen et al., Nucl. Acids Res. 33:e10 (2005); Levin and Weiss, Mol. BioSyst. 2:49-57 (2006); Bratkovic, Cell. Mol. Life Sci. 67:749-767 (2010).

For example, the technology of phage library display, and the selection by means of a phage display technique may be chosen as a method for high-throughput identification of viral protein-specific binders, because it is one of the most robust and versatile selection techniques available (Scott and Smith, Science 249:386-390 (1990); Bratkovic, Cell. Mol. Life Sci. 67:749-767(2010)). A major advantage of this technology is the coupling of genotype (i.e., the encapsulated DNA encoding the displayed protein) and phenotype (i.e., the displayed protein such as an Alphabody) which allows affinity-based selection from large libraries with millions to trillions of polypeptide variants in a relatively simple in vitro assay.

In certain particular embodiments, the methods of the present invention may further comprise the step of isolating the single-chain Alphabody library. This can be ensured by expressing the Alphabodies encoded by a nucleic acid or vector library under suitable conditions in host cells and isolating the expressed Alphabodies from the host cells and/or from the medium.

The methods of the present invention for the production of at least one single-chain Alphabody having detectable binding affinity for, or detectable in vitro activity on, a viral protein of interest at least comprise the further step of selecting from the single-chain Alphabody library at least one single-chain Alphabody having detectable binding affinity for, or detectable in vitro activity on, said viral protein of interest.

It will be understood that the selection step of the methods described herein can be performed by way of a method commonly known as a selection method or a by way of a method commonly known as a screening method. Both methods envisage the identification and subsequent isolation (i.e., the selection step) of desirable components (i.e., Alphabody library members) from an original ensemble comprising both desirable and non-desirable components (i.e., an Alphabody library). In the case of a selection method, library members will typically be isolated by a step wherein the desired property is applied to obtain the desired goal; in such case, the desired property is usually restricted to the property of a high affinity for a given viral target molecule of interest and the desired goal is usually restricted to the isolation of such high-affinity library members from the others. Such method is generally known as an affinity selection method and, in the context of the present invention, such affinity selection method will be applied to a single-chain Alphabody library for the purpose of selecting Alphabodies having a high affinity for a viral protein of interest or a subdomain or subregion thereof. Alternatively, in the case of a screening method, library members will typically be isolated by a step wherein all library members, or at least a substantial collection of library members, are individually examined with respect to a given desired property, and wherein members having such desired property are retained whereas members not having such desired property are discarded; in such case, and in the context of the present invention, desired properties may relate to either a high affinity for a viral protein of interest or a subdomain or subregion thereof, or a functional activity such as an antiviral activity, including the inhibition, reduction and/or prevention of the activity of a viral target protein of interest. Accordingly, it is submitted that the selection step of the methods of the invention may be accomplished by either an (affinity) selection technique or by an affinity-based or activity-based functional screening technique, both techniques resulting in the selection of one or more single-chain Alphabodies having beneficial (favorable, desirable, superior) affinity or activity properties compared to the non-selected Alphabodies of the single-chain Alphabody library.

Thus, in particular embodiments, the selection step comprises contacting the single-chain Alphabody library, or a mixture of single-chain Alphabody libraries, with a viral protein of interest and determining binding between the Alphabodies present in the library and the viral protein of interest. Thus this involves the step of identifying from the single-chain Alphabody library or mixture of single-chain Alphabody libraries being contacted with the target molecule of interest, the one or more single-chain Alphabodies having detectable binding affinity for the viral protein of interest. Specific binding of an Alphabody to a target molecule or protein of interest can be determined in any suitable manner known per se, including, for example biopanning, Scatchard analysis and/or competitive binding assays, such as radioimmunoassays (RIA), enzyme immunoassays (EIA) and sandwich competition assays, and the different variants thereof known in the art.

In particular embodiments, the Alphabody library is provided as a phage library and binding Alphabodies are identified by contacting the phage library with a labeled target molecule, after which binding phages are retrieved by detection or selective collection of the labeled, bound target. Typically, a biotinylated target can be used, whereby phages which display an Alphabody binding to the target are captured onto a streptavidin-coated support (e.g., magnetic beads).

In particular embodiments of the present invention, the selection steps of the methods for producing one or more single-chain Alphabodies having detectable binding affinity (as defined herein) for a viral protein of interest, may comprise the (further) enrichment of the Alphabody library, or the mixture of Alphabody libraries, for single-chain Alphabodies having detectable binding affinity for the viral protein of interest by iterative execution of the steps of contacting a viral protein of interest with a single-chain Alphabody library or with a mixture of single-chain Alphabody libraries and subsequently identifying from this library or mixture of libraries being contacted with the viral protein, the one or more single-chain Alphabodies having detectable binding affinity for the viral protein of interest.

The step of selecting a single-chain Alphabody that has detectable in vitro activity on a viral protein of interest typically comprises:
a) contacting a single-chain Alphabody library or a mixture of single-chain Alphabody libraries with a virus or cell displaying the viral protein of interest, and
b) identifying from the single-chain Alphabody library or mixture of single-chain Alphabody libraries, the one or more single-chain Alphabodies having detectable in vitro activity on the virus or cell displaying the viral protein of interest.

More particularly, the effect of the Alphabodies on the viral protein can be evaluated using a virus or viral protein-displaying cells, and a cell line susceptible to infection by this virus or susceptible to membrane fusion with the viral protein-displaying cells. Thus, the in vitro activity on a particular viral protein of interest or the efficacy of the target-specific Alphabodies may be tested using suitable methods, techniques or assays known in the art, such as suitable cell-based assays, more particularly in vitro models of viral infection.

In addition, the selection step of the methods for the production of single-chain Alphabodies that have detectable binding affinity for, or detectable in vitro activity on, viral proteins of interest may, in particular embodiments optionally comprise the step of determining the amino acid or nucleotide sequence of the one or more produced single-chain Alphabodies.

It will be understood that the selection methods described herein can also be performed as screening methods. Accordingly, the term 'selection' as used in the present description can comprise selection, screening or any suitable combination of selection and/or screening techniques.

In some cases, the methods of the present invention may further comprise the step of isolating from the single-chain Alphabody library the at least one single-chain Alphabody having detectable binding affinity for, or detectable in vitro activity on, a viral protein of interest.

The methods of the present invention may further comprise the step of amplifying the at least one single-chain Alphabody having detectable binding affinity for, or detectable in vitro activity on, a viral protein of interest. For example, a phage clone displaying a particular single-chain Alphabody, obtained from a selection step of a method of the invention, may be amplified by reinfection of a host bacteria and incubation in a growth medium.

In particular embodiments, the methods of the present invention encompass determining the sequence of the Alphabody or Alphabodies capable of binding to the viral protein of interest. Where an Alphabody polypeptide sequence, comprised in a set, collection or library of Alphabody polypeptide sequences, is displayed on a suitable cell or phage or particle, it is possible to isolate from said cell or phage or particle, the nucleotide sequence that encodes that Alphabody polypeptide sequence. In this way, the nucleotide sequence of the selected Alphabody library member(s) can be determined by a routine sequencing method.

In further particular embodiments, the methods of the invention comprise the step of expressing said nucleotide sequence(s) in a host organism under suitable conditions, so as to obtain the actual desired Alphabody polypeptide sequence(s). This step can be performed by methods known to the person skilled in the art.

In addition, the obtained Alphabody sequences having detectable binding affinity for, or detectable in vitro activity on, a viral protein of interest, may be synthesized as soluble protein construct, optionally after their sequence has been identified.

For instance, the Alphabodies obtained or obtainable by the methods of the present invention can be synthesized using recombinant or chemical synthesis methods known in the art. Also, the Alphabodies obtained or obtainable by the methods of the present invention can be produced by genetic engineering techniques. Thus, methods for synthesizing an Alphabody obtained or obtainable by the methods of the present invention may comprise transforming or infecting a host cell with a nucleic acid or a vector encoding an Alphabody sequence having detectable binding affinity for, or detectable in vitro activity on, a viral protein of interest. Accordingly, the Alphabody sequences having detectable binding affinity for, or detectable in vitro activity on, a viral protein of interest can be made by recombinant DNA methods. DNA encoding the Alphabodies can be readily synthesized using conventional procedures. Once prepared, the DNA can be introduced into expression vectors, which can then be transformed or transfected into host cells such as E. coli or any suitable expression system, in order to obtain the expression of Alphabodies in the recombinant host cells and/or in the medium in which these recombinant host cells reside.

Transformation or transfection of nucleic acids or vectors into host cells may be accomplished by a variety of means known to the person skilled in the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

Suitable host cells for the expression of the desired Alphabodies may be any eukaryotic or prokaryotic cell (e.g., bacterial cells such as E. coli, yeast cells, mammalian cells, avian cells, amphibian cells, plant cells, fish cells, and insect cells), whether located in vitro or in vivo. For example, host cells may be located in a transgenic animal.

Thus, the invention also relates to methods for the production of Alphabodies having detectable binding affinity for, or detectable in vitro activity on, a viral protein of interest comprising transforming, transfecting or infecting a host cell with nucleic acid sequences or vectors encoding such Alphabodies and expressing the Alphabodies under suitable conditions.

The methods for the production of one or more target-specific Alphabodies may further optionally comprise additional steps or methods for improving or optimizing the binding specificity and/or efficacy of the target-specific Alphabodies obtainable by the methods of the invention.

In particular embodiments, the methods for the production of one or more target-binding Alphabodies, may further be followed by steps or methods involving the rationalization of the obtained or produced Alphabody sequences. Such a sequence rationalization process may include the identification or determination of particular amino acid residues, amino acid residue positions, stretches, motifs or patterns that are conserved between or among different Alphabodies directed against (i.e., binding to) a specific target molecule of interest. Accordingly, this rationalization process can be conducted by comparing different Alphabody sequences that have been identified to bind the target protein of interest, and by identifying the sequence conservation between these sequences. Such a process can be optionally supported by or be performed by using techniques for molecular modeling and interactive ligand docking.

The particular amino acid residues, amino acid residue positions, stretches, motifs or patterns that are identified as being conserved between or among different Alphabodies against a specific target molecule of interest may be considered as contributing significantly to the binding or activity of the target-specific Alphabodies.

In particular embodiments, the process of sequence rationalization as described above may further be followed by the creation of a new library of Alphabody sequences starting from the set of different Alphabody sequences that have been identified as being specific for a target molecule of interest and that have been produced using the methods of the invention. In such embodiments, the positions where the amino acid residues, stretches, motifs or patterns are located that are conserved between or among different target-binding Alphabodies are kept constant, and the Alphabody sequences are varied in a new defined set of variegated amino acid residue positions. In this newly defined set, the variegated positions are located outside the positions where the amino acid residues, stretches, motifs or patterns are located that are conserved between or among different target-binding Alphabodies. The Alphabody libraries so obtained are referred to as 'dedicated libraries' of Alphabodies. These dedicated libraries are then again screened to identify the best target-binding Alphabody.

Accordingly, the methods for the production of one or more target-binding Alphabodies, may further, after the identification of two or more target-binding Alphabodies from a random library, comprise the steps of:
- comparing the produced Alphabody sequences that bind the target protein of interest,
- identifying the amino acid residues, amino acid residue positions, stretches, motifs or patterns that are conserved between or among these different Alphabody sequences, and:
- starting from at least one of the two or more Alphabody sequences compared, producing a dedicated library, wherein the library comprises different Alphabody sequences that are variegated in a set of amino acid positions, which are not the amino acid residues, amino acid residue positions, stretches, motifs or patterns that are conserved between or among the different target-binding Alphabody sequences,
- selecting and/or identifying from the random library those Alphabody sequences having an improved or optimized binding specificity for and/or in vitro activity on the target molecule of interest, and optionally
- isolating these Alphabody sequences having an improved or optimized binding specificity for and/or in vitro activity on the target molecule of interest.

It will be understood that the steps involved in the methods for producing a dedicated library and selecting, identifying and isolating Alphabody sequences having an improved or optimized binding specificity for and/or in vitro activity on the target molecule of interest, as described above, may be performed in a similar manner as described for the corresponding steps of the methods for producing target-binding Alphabodies.

As further described herein, the total number of amino acid residues in an Alphabody can be in the range of about 50 to about 210, depending mainly on the number of heptads per heptad repeat sequence and the length of the flexible linkers interconnecting the heptad repeat sequences. Parts, fragments, analogs or derivatives of an Alphabody, polypeptide or composition are not particularly limited as to their length and/or size, as long as such parts, fragments, analogs or derivatives still have the biological function of an Alphabody, polypeptide or composition from which they are derived and can still be used for the envisaged (pharmacological) purposes.

The application discloses polypeptides that comprise or essentially consist of at least one Alphabody that specifically binds to a viral protein. The polypeptides may comprise at least one Alphabody and optionally one or more further groups, moieties, residues optionally linked via one or more linkers.

Accordingly, a polypeptide may optionally contain one or more further groups, moieties or residues for binding to other targets or target proteins of interest. It should be clear that such further groups, residues, moieties and/or binding sites may or may not provide further functionality to the Alphabodies (and/or to the polypeptide or composition in which it is present) and may or may not modify the properties of the Alphabody. Such groups, residues, moieties or binding units may also for example be chemical groups which can be biologically and/or pharmacologically active.

These groups, moieties or residues are, in particular embodiments, linked N- or C-terminally to the Alphabody. In particular embodiments however, one or more groups, moieties or residues are linked to the body of the Alphabody, e.g. via coupling to a cysteine in an alpha-helix.

In particular embodiments, the polypeptides comprise Alphabodies that have been chemically modified. For example, such a modification may involve the introduction or linkage of one or more functional groups, residues or moieties into or onto the Alphabody. These groups, residues or moieties may confer one or more desired properties or functionalities to the Alphabody Examples of such functional groups will be clear to the skilled person and include, without limitation, a purification tag, a detection tag, a fluorescent tag, a glycan moiety, a PEG moiety.

The introduction or linkage of functional groups to an Alphabody may also have the effect of an increase in the half-life, the solubility and/or the stability of the Alphabody, or it may have the effect of a reduction of the toxicity of the Alphabody, or it may have the effect of the elimination or attenuation of any undesirable side effects of the Alphabody, and/or it may have the effect of other advantageous properties.

In particular embodiments, the polypeptides comprise Alphabodies that have been modified to specifically increase the half-life thereof, for example, by means of PEGylation, by means of the addition of a group or protein or protein domain which binds to or which is a serum protein (such as serum albumin) or, in general, by linkage of the Alphabody to a moiety that increases the half-life of the Alphabody. Typically, the polypeptides with increased half-life have a half-life that is at least twice, such as at least three times, such as at least five times, for example at least ten times or more than ten times greater than the half-life of the corresponding Alphabody lacking the said chemical modification.

A particular modification of the Alphabodies may comprise the introduction of one or more detectable labels or other signal-generating groups or moieties, depending on the intended use of the labeled Alphabody.

Yet a further particular modification may involve the introduction of a chelating group, for example to chelate one or more metals or metallic cations.

A particular modification may comprise the introduction of a functional group that is one part of a specific binding pair, such as the biotin-(strept)avidin binding pair.

For some applications, in particular for those applications in which it is intended to kill a viral particle or cell that expresses the target which the Alphabodies specifically bind to, or to reduce or slow the growth and/or proliferation of such a viral particle or cell, the Alphabodies may also be linked to a toxin or to a toxic residue or moiety.

Other potential chemical and enzymatic modifications will be clear to the skilled person.

In particular embodiments, the one or more groups, residues, moieties are linked to the Alphabody via one or more suitable linkers or spacers.

In further particular embodiments, the polypeptides comprise two or more target-specific Alphabodies. In such particular embodiments, the two or more target-specific Alphabodies may be linked (coupled, concatenated, interconnected, fused) to each other either in a direct or in an indirect way. In embodiments wherein the two or more Alphabodies are directly linked to each other, they are linked without the aid of a spacer or linker fragment or moiety. Alternatively, in embodiments wherein the two or more Alphabodies are indirectly linked to each other, they are linked via a suitable spacer or linker fragment or linker moiety.

In embodiments wherein two or more Alphabodies are directly linked, they may be produced as single-chain fusion constructs (i.e., as single-chain protein constructs wherein two or more Alphabody sequences directly follow each other in a single, contiguous amino acid sequence). Alternatively, direct linkage of Alphabodies may also be accomplished via cysteines forming a disulfide bridge between two Alphabodies (i.e., under suitable conditions, such as oxidizing conditions, two Alphabodies comprising each a free cysteine may react with each other to form a dimer wherein the constituting momomers are covalently linked through a disulfide bridge).

Alternatively, in embodiments wherein two or more Alphabodies are indirectly linked, they may be linked to each other via a suitable spacer or linker fragment or linker moiety. In such embodiments, they may also be produced as single-chain fusion constructs (i.e., as single-chain protein constructs wherein two or more Alphabody sequences follow each other in a single, contiguous amino acid sequence, but wherein the Alphabodies remain separated by the presence of a suitably chosen amino acid sequence fragment acting as a spacer fragment). Alternatively, indirect linkage of Alphabodies may also be accomplished via amino acid side groups or via the Alphabody N- or C-termini. For example, under suitably chosen conditions, two Alphabodies comprising each a free cysteine may react with a homo-bifunctional chemical compound, yielding an Alphabody dimer wherein the constituting Alphabodies are covalently cross-linked through the said homo-bifunctional compound. Analogously, one or more Alphabodies may be cross-linked through any combination of reactive side groups or termini and suitably chosen homo- or heterobifunctional chemical compounds for cross-linking of proteins.

In particular embodiments of linked Alphabodies, the two or more linked Alphabodies can have the same amino acid sequence or different amino acid sequences. The two or more linked Alphabodies can also have the same binding specificity or a different binding specificity. The two or more linked Alphabodies can also have the same binding affinity or a different binding affinity.

Suitable spacers or linkers for use in the coupling of different Alphabodies will be clear to the skilled person and may generally be any linker or spacer used in the art to link peptides and/or proteins. In particular, such a linker or spacer is suitable for constructing proteins or polypeptides that are intended for pharmaceutical use.

Some particularly suitable linkers or spacers for coupling of Alphabodies in a single-chain amino acid sequence include for example, but are not limited to, polypeptide linkers such as glycine linkers, serine linkers, mixed glycine/serine linkers, glycine- and serine-rich linkers or linkers composed of largely polar polypeptide fragments. Some particularly suitable linkers or spacers for coupling of Alphabodies by chemical cross-linking include for example, but are not limited to, homo-bifunctional chemical cross-linking compounds such as glutaraldehyde, imidoesters such as dimethyl adipimidate (DMA), dimethyl suberimidate (DMS) and dimethyl pimelimidate (DMP) or N-hydroxysuccinimide (NHS) esters such as dithiobis(succinimidylpropionate) (DSP) and dithiobis(sulfosuccinimidylpropionate) (DTSSP). Examples of hetero-bifunctional reagents for cross-linking include, but are not limited to, cross-linkers with one amine-reactive end and a sulfhydryl-reactive moiety at the other end, or with a NHS ester at one end and an SH-reactive group (e.g., a maleimide or pyridyl disulfide) at the other end.

A polypeptide linker or spacer for usage in single-chain concatenated Alphabody constructs may be any suitable (e.g., glycine-rich) amino acid sequence having a length between 1 and 50 amino acids, such as between 1 and 30, and in particular between 1 and 10 amino acid residues. It should be clear that the length, the degree of flexibility and/or other properties of the spacer(s) may have some influence on the properties of the final polypeptide, including but not limited to the affinity, specificity or avidity for a viral protein of interest, or for one or more other target proteins of interest. It should be clear that when two or more spacers are used in the polypeptides, these spacers may be the same or different. In the context and disclosure of the present invention, the person skilled in the art will be able to determine the optimal spacers for the purpose of coupling Alphabodies without any undue experimental burden.

The linked Alphabody polypeptides can generally be prepared by a method which comprises at least one step of suitably linking the one or more Alphabodies to the one or more further groups, residues, moieties and/or other Alphabodies, optionally via the one or more suitable linkers, so as to provide a polypeptide.

Also, the polypeptides can be produced by methods at least comprising the steps of: (i) expressing, in a suitable host cell or expression system, the polypeptide, and (ii) isolating and/or purifying the polypeptide. Techniques for performing the above steps are known to the person skilled in the art.

The present invention also encompasses parts, fragments, analogs, mutants, variants, and/or derivatives of the Alphabodies and polypeptides and/or polypeptides comprising or essentially consisting of one or more of such parts, fragments, analogs, mutants, variants, and/or derivatives. In particular embodiments, these parts, fragments, analogs, mutants, variants, and/or derivatives are capable of binding to a viral protein of interest. Most particularly the binding affinity of the part, fragment, analog, mutant, variant, and/or derivative of the Alphabodies and polypeptides bind to the viral protein of interest with a binding affinity which is comparable or increased compared to the Alphabody from which it is derived. In particular embodiments, the parts, fragments, analogs, mutants, variants, and/or derivatives of the Alphabodies and polypeptides are suitable for the prophylactic, therapeutic and/or diagnostic purposes envisaged herein. Such parts, fragments, analogs, mutants, variants, and/or derivatives according to the invention are still capable of specifically binding to a viral protein.

It should be noted that the Alphabodies, polypeptides or compositions (or of the nucleotide sequences used to express them) are not naturally occurring proteins (or nucleotide sequences). Furthermore, the present invention is also not limited as to the way that the Alphabodies, polypeptides or nucleotide sequences have been generated or obtained. Thus, the Alphabodies may be synthetic or semi-synthetic amino acid sequences, polypeptides or proteins.

The Alphabodies, polypeptides and compositions provided by the invention can be in essentially isolated form (as defined herein), or alternatively can form part of a polypeptide or composition, which may comprise or essentially consist of at least one Alphabody and which may optionally further comprise one or more other groups, moieties or residues (all optionally linked via one or more suitable linkers or spacers).

It will be appreciated based on the disclosure herein that for prophylactic, therapeutic and/or diagnostic applications, the Alphabodies, polypeptides and compositions will in principle be directed against or specifically bind to viral proteins, of human viruses. However, where the Alphabodies, polypeptides and compositions are intended for veterinary purposes, they will be directed against or specifically bind to viral proteins of viruses that are able to infect and/or reproduce themselves in the species intended to be treated, or they will be at least cross-reactive with viral proteins of viruses that are able to infect and/or reproduce themselves in the species to be treated. Accordingly, Alphabodies, polypeptides and compositions that specifically bind to viral proteins of viruses that are able to infect and/or reproduce themselves in one subject species may or may not show cross-reactivity with viral proteins of viruses that are able to infect and/or reproduce themselves in one or more other subject species. Of course it is envisaged that, in the context of the development of Alphabodies for use in humans or animals, Alphabodies may be developed which bind to viral proteins of viruses that are able to infect and/or reproduce themselves in another species than that which is to be treated for use in research and laboratory testing.

It is also expected that the Alphabodies and polypeptides may bind to some naturally occurring or synthetic analogs, variants, mutants, alleles, parts and fragments of a viral protein. More particularly, it is expected that the Alphabodies and polypeptides will bind to at least to those analogs, variants, mutants, alleles, parts and fragments of a viral protein that (still) contain the binding site, part or domain of the (natural/wild-type) viral protein and/or the viral protein to which those Alphabodies and polypeptides bind.

In particular embodiments the Alphabodies, polypeptides and compositions that specifically bind to a viral protein of interest do not show cross-reactivity with a naturally occurring protein other than the target protein of interest, most particularly do not show cross-reactivity with a protein naturally occurring in mammals.

The application further discloses nucleic acid sequences encoding single-chain Alphabodies, which are obtainable by the methods according to the invention as well as vectors and host cells comprising such nucleic acid sequences.

In a further aspect, the present invention provides nucleic acid sequences encoding the Alphabodies or the polypeptides (or suitable fragments thereof). These nucleic acid sequences are also referred to herein as nucleic acid sequences and can also be in the form of a vector or a genetic construct or polynucleotide. The nucleic acid sequences may be synthetic or semi-synthetic sequences, nucleotide sequences that have been isolated from a library (and in particular, an expression library), nucleotide sequences that have been prepared by PCR using overlapping primers, or nucleotide sequences that have been prepared using techniques for DNA synthesis known per se.

The genetic constructs may be DNA or RNA, and are preferably double-stranded DNA. The genetic constructs may also be in a form suitable for transformation of the intended host cell or host organism or in a form suitable for integration into the genomic DNA of the intended host cell or in a form suitable for independent replication, maintenance and/or inheritance in the intended host organism. For instance, the genetic constructs may be in the form of a vector, such as for example a plasmid, cosmid, YAC, a viral vector or transposon. In particular, the vector may be an expression vector, i.e., a vector that can provide for expression in vitro and/or in vivo (e.g. in a suitable host cell, host organism and/or expression system).

In a further aspect, the invention provides vectors comprising nucleic acids encoding single-chain Alphabodies, which are obtainable by the methods according to the invention.

The application further discloses host cells comprising nucleic acids encoding single-chain Alphabodies, which are obtainable by the methods according to the invention or vectors comprising these nucleic acids. Accordingly, a particular embodiment is a host cell transfected or transformed with a vector comprising the nucleic acid sequence encoding the Alphabodies obtainable by the methods of the invention and which is capable of expressing the Alphabodies. Suitable examples of hosts or host cells for expression of the Alphabodies or polypeptides will be clear to the skilled person and include any suitable eukaryotic or prokaryotic cell (e.g., bacterial cells such as E. coli, yeast cells, mammalian cells, avian cells, amphibian cells, plant cells, fish cells, and insect cells), whether located in vitro or in vivo.

The application further discloses the use of the Alphabodies and polypeptides to detect a viral protein of interest in vitro or in vivo.

In particular embodiments, the Alphabodies and polypeptides of the present invention comprise a label or other signal-generating moiety. Suitable labels and techniques for attaching labels on Alphabodies are known in the art. These include, but are not limited to, fluorescent labels, phosphorescent labels, chemiluminescent labels, bioluminescent labels, radio-isotopes, metals, metal chelates, metallic cations, chromophores and enzymes.

Such labeled Alphabodies and polypeptides may for example be used for in vitro, in vivo or in situ assays (including immunoassays known per se such as ELISA, RIA, EIA and other 'sandwich assays', etc.) as well as in vivo diagnostic and imaging purposes, depending on the choice of the specific label.

In further particular embodiments, the invention relates to the use of the target-specific Alphabodies and polypeptides for drug delivery. More particularly, it can be envisaged that the Alphabodies, as a result of their specific binding to a viral protein, such as a viral fusion protein, can be designed to deliver, upon binding to their viral protein target (typically naturally located at the surface of a virus), an antiviral compound.

The application further discloses the use of the Alphabodies and polypeptides to inhibit, reduce and/or prevent the biological activity of a viral protein. Most particularly, it is envisaged that the Alphabodies and polypeptides affect the interaction between a viral protein and a natural binding partner, for example, a specific receptor, located on a target cell or between different domains, subdomains, regions, subregions or parts of that viral protein. The Alphabodies, polypeptides and pharmaceutical compositions disclosed herein can thus be used to inhibit, reduce and/or prevent a biological activity of a viral protein, as can be measured using a suitable in vitro, cellular or in vivo assay. The Alphabodies, polypeptides and pharmaceutical compositions can also be used to inhibit, reduce and/or prevent one or more biological or physiological mechanisms, effects, responses, functions pathways or activities in which a viral protein is involved. Such an action of the Alphabody, polypeptide or composition according to the invention as an antagonist, in the broadest possible sense, may be determined in any suitable manner and/or using any suitable (in vitro and usually cellular or in vivo) assay known in the art, depending on the type of inhibition, reduction and/or prevention of the said one or more biological or physiological mechanisms, effects, responses, functional pathways or activities in which the said viral protein is involved. Non-limiting examples of such types of functional effects include (i) the prevention of attachment to cellular receptors on specific, dedicated target cells, (ii) the prevention of interaction with the glycocalyx of target cells, (iii) the formation of a steric block at the surface of a virion or a viral protein-displaying cell, (iv) the arrest of a viral protein in its native state through blockage of conformational changes that are required for membrane fusion, (v) the arrest of a viral protein in a conformational or mechanistic state that is intermediate to the native and postfusion states, (vi) the irreversible functional deactivation of a viral protein prior to attachment to a target cell, and wherein said deactivation is further characterized by the inability of said viral protein to recover membrane fusion activity even after removal of the antiviral Alphabody, polypeptide or composition according to the invention.

In view of the ability of the Alphabodies and polypeptides disclosed herein to inhibit viral protein functions in vivo, the application also discloses pharmaceutical compositions comprising one or more Alphabodies, polypeptides and/or nucleic acid sequences, which are obtainable by the methods according to the invention and optionally at least one pharmaceutically acceptable carrier. According to certain particular embodiments, the pharmaceutical compositions may further optionally comprise at least one other pharmaceutically active compound.

The pharmaceutical compositions disclosed herein can be used in the diagnosis, prevention and/or treatment of diseases and disorders associated with viral diseases, more particularly with viral infection, viral entry or viral fusion being mediated by a viral protein.

In particular, the application discloses pharmaceutical compositions comprising Alphabodies and polypeptides that are suitable for prophylactic, therapeutic and/or diagnostic use in a warm-blooded animal, and in particular in a mammal, and more in particular in a human being.

The present application also discloses pharmaceutical compositions comprising Alphabodies and polypeptides that can be used for veterinary purposes in the prevention and/or treatment of one or more diseases, disorders or conditions associated with and/or mediated by a viral protein.

Generally, for pharmaceutical use, the polypeptides may be formulated as a pharmaceutical preparation or compositions comprising at least one Alphabody or polypeptide and at least one pharmaceutically acceptable carrier, diluent or excipient and/or adjuvant, and optionally one or more further pharmaceutically active polypeptides and/or compounds. Such a formulation may be suitable for oral, parenteral, topical administration or for administration by inhalation. Thus, the Alphabodies, or polypeptides and/or the compositions comprising the same can for example be administered orally, intraperitoneally, intravenously, subcutaneously, intramuscularly, transdermally, topically, by means of a suppository, by inhalation, again depending on the specific pharmaceutical formulation or composition to be used. The clinician will be able to select a suitable route of administration and a suitable pharmaceutical formulation or composition to be used in such administration.

The pharmaceutical compositions may also contain suitable binders, disintegrating agents, sweetening agents or flavoring agents. Tablets, pills, or capsules may be coated for instance with gelatin, wax or sugar and the like. In addition, the Alphabodies and polypeptides may be incorporated into sustained-release preparations and devices.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form must be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. Antibacterial and antifungal agents and the like can optionally be added.

Useful dosages of the Alphabodies and polypeptides can be determined by comparing their in vitro activity, and in vivo activity in animal models. Methods for the extrapolation of effective dosages in mice, and other animals, to humans are known to the skilled person.

The amount of the Alphabodies and polypeptides required for use in prophylaxis and/or treatment may vary not only with the particular Alphabody or polypeptide selected but also with the route of administration, the nature of the condition being treated and the age and condition of the patient and will be ultimately at the discretion of the attendant physician or clinician.

The Alphabodies or polypeptides and/or the compositions comprising the same are administered according to a regimen of treatment that is suitable for preventing and/or treating the disease or disorder to be prevented or treated. The clinician will generally be able to determine a suitable treatment regimen. Generally, the treatment regimen will comprise the administration of one or more Alphabodies and/or polypeptides, or of one or more compositions comprising the same, in one or more pharmaceutically effective amounts or doses.

The desired dose may conveniently be presented in a single dose or as divided doses (which can again be sub-dosed) administered at appropriate intervals. An administration regimen could include long-term (i.e., at least two weeks, and for example several months or years) or daily treatment.

The Alphabodies and polypeptides disclosed herein will be administered in an amount which will be determined by the medical practitioner based inter alia on the severity of the condition and the patient to be treated. Typically, for each disease indication an optimal dosage will be determined specifying the amount to be administered per kg body weight per day, either continuously (e.g. by infusion), as a single daily dose or as multiple divided doses during the day. The clinician will generally be able to determine a suitable daily dose, depending on the factors mentioned herein. It will also be clear that in specific cases, the clinician may choose to deviate from these amounts, for example on the basis of the factors cited above and his expert judgment.

In particular, the Alphabodies and polypeptides may be used in combination with other pharmaceutically active compounds or principles that are or can be used for the prevention and/or treatment of the diseases and disorders cited herein, as a result of which a synergistic effect may or may not be obtained. Examples of such compounds and principles, as well as routes, methods and pharmaceutical formulations or compositions for administering them will be clear to the clinician.

The application further discloses the use of Alphabodies or polypeptides that specifically bind to a viral protein, most particularly to a viral fusion protein, for the preparation of a medicament for the prevention and/or treatment of at least one viral protein-mediated disease and/or disorder in which said viral protein is involved. Accordingly, the invention provides Alphabodies, polypeptides and pharmaceutical compositions specifically binding to a viral protein for use in the prevention and/or treatment of at least one viral protein-mediated disease and/or disorder in which said viral protein are involved. In particular embodiments, the present invention also provides methods for the prevention and/or treatment of at least one viral protein-mediated disease and/or disorder, comprising administering to a subject in need thereof, a pharmaceutically active amount of one or more Alphabodies, polypeptides and/or pharmaceutical compositions. In particular, the pharmaceutically active amount may be an amount that is sufficient (to create a level of the Alphabody or polypeptide in circulation) to inhibit, prevent or decrease the biological or physiological mechanisms, effects, responses, functional pathways or activities in which viral proteins are involved.

The subject or patient to be treated with the Alphabodies or polypeptides may be any warm-blooded animal, but is in particular a mammal, and more in particular a human suffering from, or at risk of, diseases and disorders in which the viral protein to which the Alphabodies or polypeptides specifically bind to are involved.

'Viral diseases (and disorders)', 'viral infections', or 'diseases and disorders in which (a) viral protein(s) is/are involved' as used in the context of the present invention can be defined as diseases and disorders that are caused by one or more viruses. In particular embodiments, viral diseases are diseases that can be prevented and/or treated by suitably administering to a subject in need thereof (i.e. having the disease or disorder or at least one symptom thereof and/or at risk of attracting or developing the disease or disorder) of either an Alphabody, polypeptide or composition.

In particular embodiments, the Alphabody or polypeptide binds to a viral fusion protein and the viral disease or viral infection is a disease caused by a virus characterized by the presence of a viral fusion protein.

Examples of viral diseases or viral infections will be clear to the skilled person based on the disclosure herein, and for example include the following diseases and disorders (caused by the following viruses): AIDS (caused by HIV), AIDS Related Complex (caused by HIV), Aseptic meningitis (caused by HSV-2), Bronchiolitis (caused by e.g. RSV), California encephalitis (caused by California encephalitis virus), Chickenpox (caused by Varicella zoster virus), Colorado tick fever (caused by Colorado tick fever virus), Common cold (caused by e.g. RSV or Parainfluenza virus), Conjunctivitis (caused by e.g. Herpes simplex virus), Cowpox (caused by vaccinia virus), Croup (caused by e.g. parainfluenza viruses 1 to 3), Cytomegalovirus Infection (caused by cytomegalovirus), Dengue fever (caused by dengue virus), Eastern equine encephalitis (caused by EEE virus), Ebola hemorrhagic fever (caused by Ebola virus), encephalitis and chronic pneumonitis in sheep (caused by Visna virus), encephalitis (caused by Semliki Forest virus), Gingivostomatitis (caused by HSV-I), Hantavirus hemorrhagic fever/Hantaan-Korean hemorrhagic fever (caused by Hantavirus), Hepatitis (caused by Hepatitis virus), Genital herpes (caused by HSV-2), Herpes labialis (caused by HSV-I), neonatal herpes (caused by HSV-2), Genital HSV (caused by Herpes simplex virus), Infectious mononucleosis (caused by e.g. Epstein-Barr virus), Influenza (Flu) (caused by influenza viruses A, B and C), Japanese encephalitis virus (caused by JEE virus), Keratoconjunctivitis (caused by HSV-I), Lassa fever, Leukemia and lymphoma (caused by e.g. Human T cell leukemia virus or Moloney murine leukemia virus), Lower respiratory tract infections (caused by e.g. RSV or Sendai virus), Measles (caused by rubeola virus), Marburg hemorrhagic fever (caused by Marburg virus), Molluscum contagiosum (caused by Molluscum), Mononucleosis-like syndrome (caused by CMV), mumps (caused by mumps virus), Newcastle disease (caused by avian paramoxyvirus 1), Norovirus, Orf (caused by Orf virus), Pharyngitis (caused by e.g. RSV, Influenza virus, Parainfluenza virus and Epstein-Barr virus), Pneumonia (viral) (caused by e.g. RSV or CMV), Progressive multifocal leukencephalopathy, Rabies (caused by Rabies virus), Roseola (caused by HHV-6), Rubella (caused by rubivirus), SARS (caused by a human coronavirus), Shingles (caused by Varicella zoster virus), Smallpox (caused by Variola virus), St. Louis encephalitis (caused by SLE virus), Strep Throat (caused by e.g. RSV, Influenza viruses, Parainfluenza virus, Epstein-Barr virus), Sindbis fever (Sindbis virus), Temporal lobe encephalitis (caused by HSV-I), Urethritis (caused by Herpes simplex virus), Vesicular stomatitis (caused by vesicular stomatitis virus), Viral encephalitis, Viral gastroenteritis, Viral meningitis, Viral pneumonia, Western equine encephalitis (caused by WEE virus), West Nile disease, Yellow fever (caused by Yellow Fever virus), and Zoster (caused by Varicella zoster virus).

The efficacy of the Alphabodies and polypeptides, and of compositions comprising the same, can be tested using any suitable in vitro assay, cell-based assay, in vivo assay and/or animal model known per se, or any combination thereof, depending on the specific disease or disorder involved. Suitable assays and animal models will be clear to the skilled person, and for example include those listed in McMahon et al., Curr. Opin. Infect. Dis. 22:574-582 (2009), as well as the assays and animal models used in the experimental part below and in the prior art cited herein. Depending on the viral protein(s) involved, the skilled person will generally be able to select a suitable in vitro assay, cellular assay or animal model to test the Alphabodies and polypeptides for their capacity to affect the activity of these viral proteins, and/or the biological mechanisms in which these are involved; and for their therapeutic and/or prophylactic effect in respect of one or more diseases and disorders that are associated with a viral protein.

### BRIEF DESCRIPTION OF THE DRAWINGS

The invention will now be further described by means of the following non-limiting **Examples and Figures,** in which the **FIGURES** show:

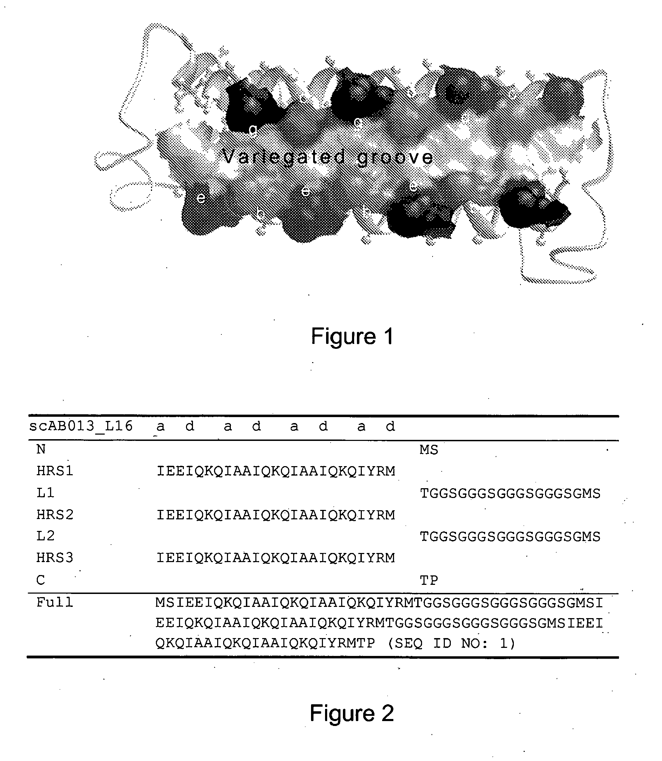

**Figure 1****.** Illustration of Alphabody groove. The drawing shows a simplified representation of a single-chain Alphabody of the same dimensions as the 'scAB013_L16' Alphabody described herein. All amino acid residues are depicted as alanines (C-beta atoms only). The backbone trace is shown in ribbon representation. Primary, secondary and core groove positions (as defined herein) are surface-rendered to illustrate the location and shape of a groove. The c- and g-residues from one alpha-helix (the A-helix in library scLib_AC11) and the b- and e-residues from a second alpha-helix (the C-helix in library scLib_AC11) are labeled accordingly.
**Figure 2****.** Illustration of the amino acid sequences of single-chain Alphabody scAB013 L16. The full amino acid sequence in single-letter code is listed at the bottom, to the right of the label 'Full'. Specific segments within the same sequence are also shown on top, to facilitate identification of N- and C-terminal flanking segments (labeled 'N' and 'C', respectively), linker segments (labeled 'L1' and 'L2', respectively) and the actual heptad repeat sequences (labeled 'HRS1', 'HRS2' and 'HRS3'). Heptad a- and d-positions are provided at the top row to facilitate their identification within the heptad repeat sequences.

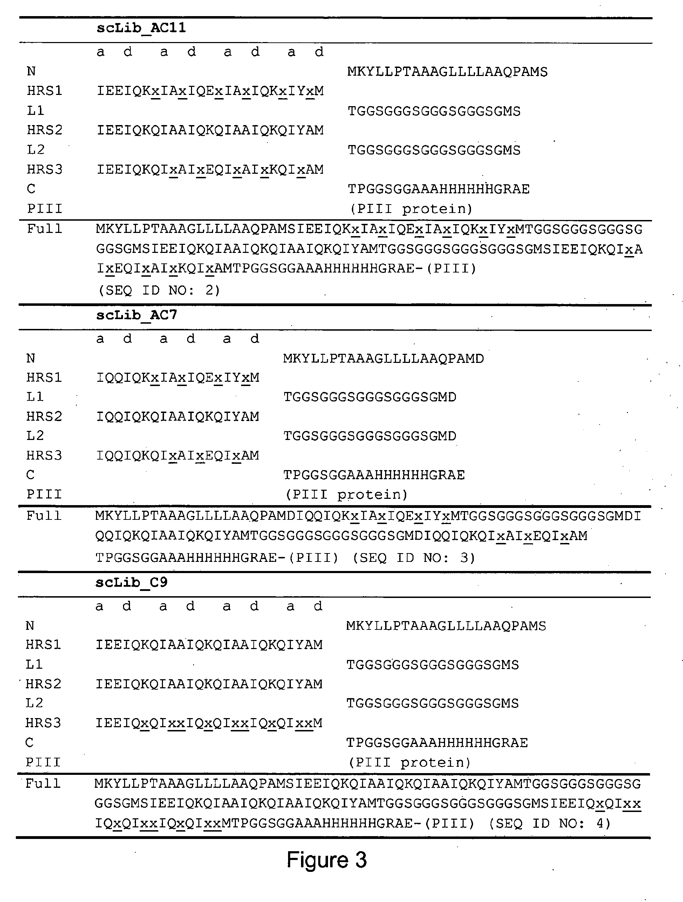

**Figure 3****.** Illustration of single-chain Alphabody libraries. Three Alphabody libraries are shown, each being C-terminally connected to the N-terminus of the phage coat protein pIII. The name of each library is indicated at the top row of each of the three panels ('scLib_AC11', 'scLib_C9' and 'scLib_C7'). Their full amino acid sequences are listed (in single-letter notation) at the bottom of each table panel, to the right of the label 'Full'. The symbol 'x' is used at positions that are randomized. 'PIII' denotes the phage pIII coat protein. Specific segments within the same sequences are also shown on top, to facilitate identification of N- and C-terminal flanking segments (labeled 'N' and 'C', respectively), linker segments (labeled 'L1' and 'L2', respectively) and the actual heptad repeat sequences (labeled 'HRS1', 'HRS2' and 'HRS3'). Heptad a- and d-positions are provided at the top row to facilitate their identification within the heptad repeat sequences.

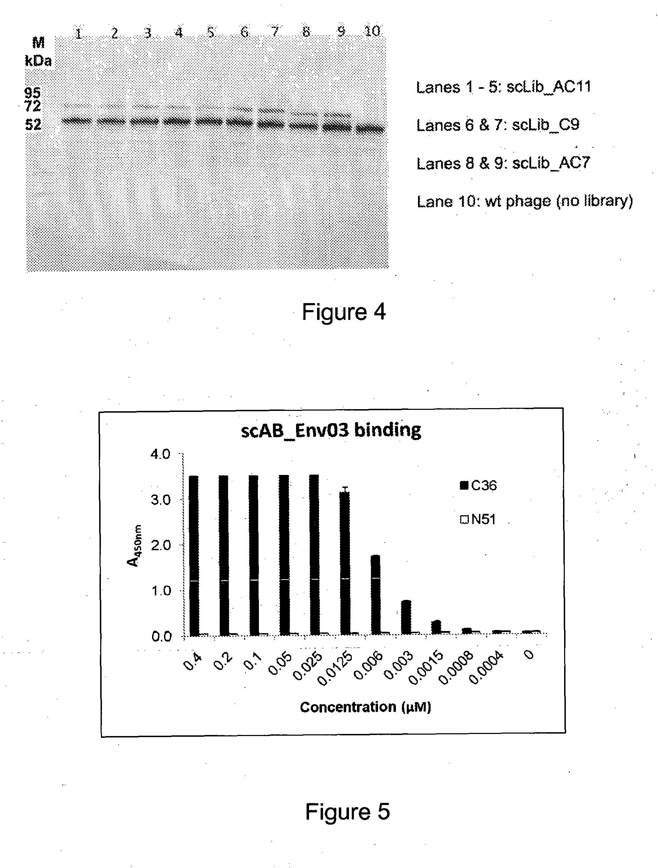

**Figure 4****.** Western-Blot analysis of Alphabody library phages. In each lane, 2x10¹¹ phages were applied. Several batches of phages from each library were tested. Samples were blotted onto nitrocellulose membrane after SDS-PAGE in reducing conditions. The presence of the fusion product was demonstrated by using a mouse anti-gplll antibody followed by an AP-anti-mouse conjugate and the blot was developed by adding the NBT-BCIP substrate.
**Figure 5****.** scAB_Env03 binding to bL4_C36. Binding of scAB_Env03 to the C-peptide 'bL4_C36' and to the negative control N-peptide 'bL4_N51' was tested in by ELISA. Plates were coated with 10 µg/ml neutravidin, 150 µl per well, incubated overnight at 4 °C and washed 3 times with PBS/0.05% Tween-20. Blocking was carried out with PBS/2% skim milk, 200 µl/well, incubated for 1 h at room temperature (RT) and washed 3 times with PBS/0.05% Tween-20. Target peptides were immobilized at a concentration of 100 nM in PBS, 100 µl per well, incubated for 1 h at RT, and washed 4 times with PBS/0.05% Tween-20. Serially diluted alpha-body in PBS/0.1% SM, 100 µl per well, was added to different wells, incubated for 2 h at RT, and washed 4 times with PBS/0.05% Tween-20. Detection of bound alpha-body was performed with Penta-His-HRP antibody (Qiagen) 1/2000 in PBS/0.1% SM, 100 µl per well, incubated for 1 h at RT, and washed 4 times with PBS/0.05% Tween-20. Coloring was performed with substrate TMB, 100 µl per well, incubated until visible coloring was observed, followed by addition of 1 N sulfuric acid, 100 µl per well. Plates were spectrophotometrically analyzed at 450 nm. Binding of each Alphabody was tested in triplicate. The A₄₅₀ₙₘ signals (mean values plus/minus standard deviations of three measurements) are plotted as a function of Alphabody concentrations. Black bars labeled 'C36' show binding to immobilized bL4_C36; white bars labeled 'N51' show (non)binding to immobilized bL4_N51.

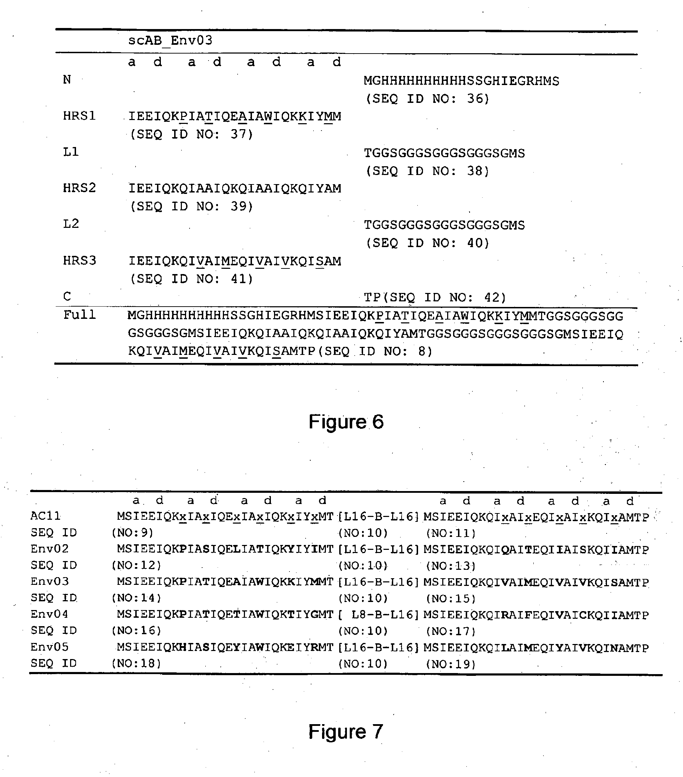

**Figure 6****.** scAB_Env03 sequence. The amino acid sequence of single-chain Alphabody scAB_Env03 is depicted in the same format as the Alphabody library constructs of Figure 3.
**Figure 7****.** Aligned single-chain Alphabody sequences. The sequences of Alphabodies scAB_Env02 ('Env02'), scAB_Env03 ('Env03'), scAB_Env04 ('Env04') and scAB_Env05 ('Env05') are aligned with the library sequence scLib_AC11 ('AC11'). Only the sequences of the A- and C-helices are written in full. The linker fragment sequences and B-helix sequence are abbreviated as 'L16' and 'B', respectively and are the same as in the library sequence (Figure 3). The first linker segment of the scAB_Env04 construct was found to be only 8 residues long ('L8'), presumably due to an unintended deletion in the library construction.

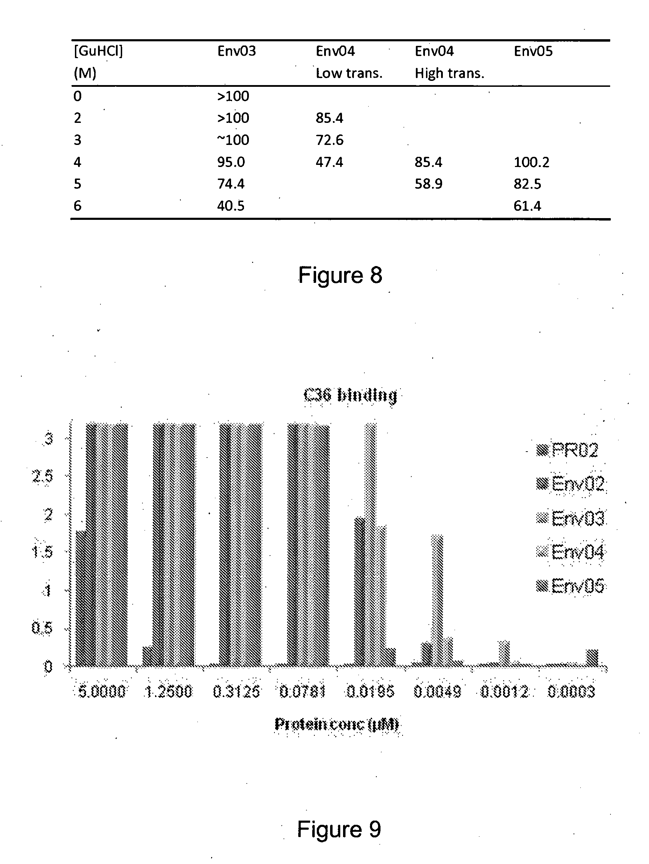

**Figure 8****.** Transition temperatures from CD thermal unfolding in different concentrations of denaturant. The transition temperatures (Tm) for scAB_Env03 ('Env03'), scAB_Env04 ('Env04') and scAB_Env05 ('Env05') were derived from the thermal unfolding curves measured by the circular dichroism (CD) signal at 222 nm, and recorded in the presence of different concentrations of GuHCl, as indicated. For scAB_Env04 at 4 M GuHCl, a two-step transition ('low trans.' and 'high trans.') was observed. At lower (or higher) concentrations of GuHCl, a single low (respectively high) transition was observed.
**Figure 9****.** ELISA results for different scAB_Env constructs. scAB_Env02 to -05 were tested for their binding to bL4_C36, immobilized on neutravidin-coated plates. The same ELISA protocol was applied as in Figure 5. 'PR02' refers to the control Alphabody scAB_PR02 which was selected from an earlier biopanning procedure on an unrelated target.

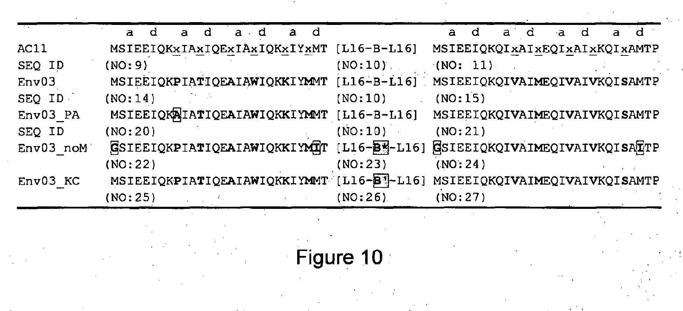

**Figure 10****.** Aligned single-chain Alphabody sequences. The sequences of Alphabodies scAB_Env03 ('Env03'), scAB_Env03_PA ('Env03_PA'), scAB_Env03 noM ('Env03_noM') and scAB_Env03_KC ('Env03_KC') are aligned with the library sequence scLib_AC11 ('AC11'). The same notation is used as in Figure 7. Mutations with respect to the parental construct scAB_Env03 are shaded. The sequence of fragment labeled B* is GSIEEIQKQIAAIQKQIAAIQKQIYAIT (SEQ ID NO: 28). The sequence of the. fragment labeled B' is MSIEEIQKQIAAIQCQIAAIQKQIYAMT (SEQ ID NO:29).

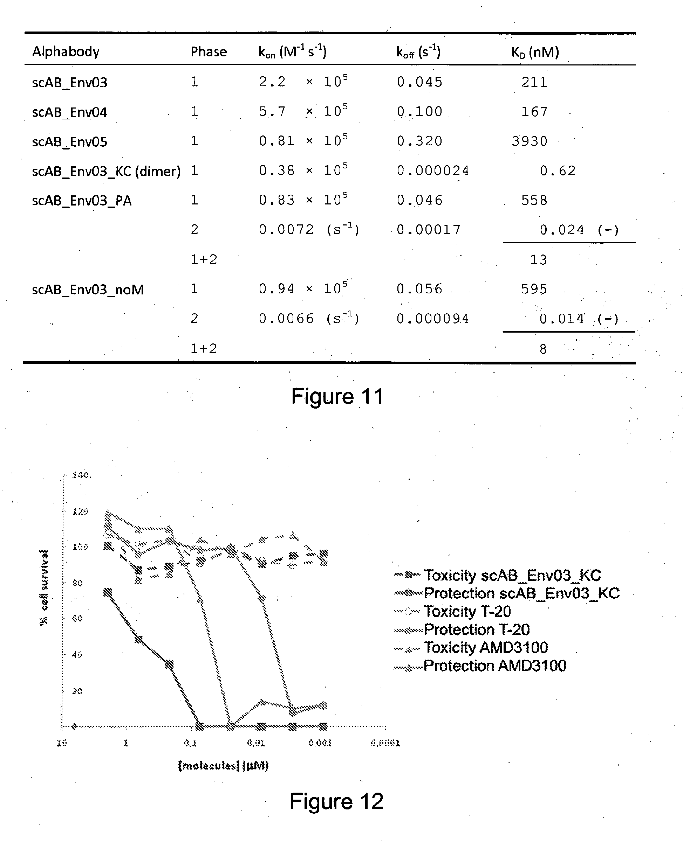

**Figure 11****.** Summary of kinetic data. The on- and off-rate constants ('kₒₙ' and 'k_{off}', respectively) and derived dissociation constants ('K_{D}') are provided for different constructs, as indicated. The kinetic parameters were obtained by surface plasmon resonance (SPR) on bL4_C36 immobilized on a streptavidin sensor chip. An irrelevant biotinylated reference peptide was immobilized on another flow cell of the same sensor chip under the same conditions as bL4_C36. Two-fold dilutions of Alphabodies were made, starting at 250 nM to 7.8 nM. They were injected at a flow rate of 30 µl/min during 2 min. Dissociation was monitored for 15 min. For all sensorgrams, the signal obtained from the control cell was subtracted from the signal obtained on the relevant cell comprising bL4_C36. Kinetic data analysis was performed with the BIAEVALUATION 4.1 software using a Langmuir model (where appropriate) or using a two-step kinetic binding model if a clear sign of such binding was observed (scAB_Env03_PA and scAB_Env03_noM).
**Figure 12****.** Inhibitory capacity of scAB_Env03_KC. The single-chain Alphabody scAB_Env03_KC was tested on its HIV-inhibitory capacity in a standard MTT assay as described in EXAMPLE 3, along with positive control inhibitors T-20 and AMD3100. The HIV inhibitory capacity of the different molecules is expressed as the percentage of cell survival after 5 days of infection with 100 TCID50/ml of HXB2 laboratory adapted reference strain. When the molecules completely protect the cells from HIV infection, a cell survival percentage of 100% is indicated. The toxicity of the molecules was assessed in the same assay by incubating the cells in the presence of the serial dilutions of molecules but in absence of virus. When the tested molecule is not toxic, a 100% cell survival is indicated.

### EXAMPLES

### EXAMPLE 1. Generation of single-chain Alphabody library.

The present example demonstrates that a single-chain Alphabody library can be obtained which is well-displayed on phage and which is potentially useful for obtaining single-chain Alphabody sequences that bind to a viral protein of interest.

A single-chain Alphabody random library was designed starting from the annotated amino acid sequence and a 3-D model of a reference Alphabody denoted 'scAB013_L16'. A simplified 3-D model of this reference Alphabody is illustrated in Figure 1. The amino acid sequence of scAB013_L16 is also provided herein as SEQ ID No: 1. The sequence is further shown in Figure 2, wherein the conventional heptad core positions are indicated as well.

An Alphabody groove is formed by two spatially adjacent alpha-helices of a folded Alphabody protein (Figure 1). Since there are three alpha-helices per Alphabody, there are in principle three candidate grooves which can be randomized. The said 3-D model was inspected first to select the most suitable groove for randomization. It was decided to select the groove between the first alpha-helix ('A-helix') and third alpha-helix ('C-helix'), which run parallel in the 3-D model. Next, the model was further inspected to identify the most suitable amino acid residue positions to be randomized (variegated, varied) in each alpha-helix. It was observed that the groove is actually formed by residues located at heptad c- and g-positions in the A-helix and at heptad b- and e-positions in the C-helix. The g- and e-positions were found to contribute the most (i.e., most directly) to the groove, and are therefore denoted 'primary groove positions'. The c- and b-positions are located somewhat remotely from the middle of the groove, and are therefore denoted 'secondary groove positions'. In addition to these primary and secondary groove positions, the bottom of a groove is formed by some core (a- and d-) positions; in particular, the model showed that core d-positions of the A-helix and a-positions of the C-helix might contribute to the shape of the groove as well, especially if the primary groove positions are occupied by tiny amino acid residues such as glycine, alanine or serine. Such core a- and d-positions which may conditionally contribute to the shape of a groove are herein denoted 'core groove positions'.

Figure 1 shows that there are 3 primary e- and also 3 primary g-positions within the coiled coil part of the scAB013_L16 Alphabody model. It is further seen that there are 4 band 4 c-positions at secondary groove positions. Further, there are 4 core d- and 4 core e-positions which may potentially contribute to the groove. Thus, there are in total 22 positions which can influence the shape of a groove when being variegated. When all 22 would be fully randomized into the 20 natural amino acid residues, this would correspond to a sequence space (i.e., the total number of possible combinations) of 20²² or about 4 x 10²⁸ distinct sequences. Clearly such huge libraries cannot be made in a form wherein all different sequences are physically present (i.e., such library cannot be 'complete'). Consequently, and if the envisaged library is aimed to be complete (or nearly complete), then the number of variable positions should be drastically reduced.

It was therefore decided not to vary any of the core groove positions. This decision was further motivated by the (avoidance of) risks associated with mutating core positions in a coiled coil: many such substitutions would be detrimental for the stability and/or correct folding of the respective Alphabody constructs. Further, it was also decided not to vary two secondary groove positions. In particular, the first c-position in the A-helix and the first b-position in the C-helix were kept constant. Finally, the first primary groove e-position in the C-helix was also kept fixed as well. This resulted in the selection of 11 variegated positions within the context of the reference Alphabody scAB013_L16. The theoretic sequence space of such library, when fully randomized, is thus 20¹¹ or about 2 x 10¹⁴ distinct sequences.

In addition to the variegated positions, two other types of modifications to the reference Alphabody scAB013_L16 were made. First, two lysine to glutamic acid mutations were introduced, i.e., one such mutation at the f-position of the second heptad in each of helices A and C. Second, two arginine to alanine mutations were introduced, i.e., one such mutation at the c-position of the fourth heptad in each of helices B and C. The sequence of this modified single-chain Alphabody, wherein positions to be variegated are indicated by 'x'-symbols, is shown in Figure 3. This sequence is also provided as SEQ ID No: 2. The single-chain Alphabody library that was constructed on the basis of this design is hereinafter referred to as 'scLib_AC11'. Since all variegated positions are located in an Alphabody groove, this library is also referred to as a 'groove library'.

A second single-chain Alphabody groove library was designed starting from a 3-D model of a smaller Alphabody reference construct denoted 'scAB140_L14'. The latter essentially corresponds to the scAB013_L16 construct wherein the third heptad in each of the alpha-helices is deleted, the glycine/serine linker sequences are reduced from 16 to 14 residues, and the N-terminal alpha-helix capping residues are substituted by an alternative, less negatively charged, motif. Apart from these differences, exactly the same choices with respect to primary, secondary and core groove positions to be variegated were made when designing the library. In view of the deletion of one heptad unit in each of the helices, this library comprises only 7 variable residue positions. The theoretic sequence space for full randomization is therefore 20⁷ or about 10⁹, which should guarantee near-completeness of the actual produced library. The sequence of this single-chain Alphabody groove library, denoted 'scLib_AC7', is shown in Figure 3. This sequence is also provided as SEQ ID No: 3.

A third single-chain Alphabody library was designed to explore the potential of generating Alphabodies that bind to their target via a surface-exposed area on a single alpha-helix. In other words, the purpose of this design was to generate an Alphabody 'helix library' (as opposed to the scLib_AC11 and scLib_AC7 libraries which are groove libraries). The 3-D model of scAB013_L16 was again used as the template structure for guiding the selection of positions to be variegated. It was decided to select the C-helix in this structure as the one to be variegated. Further inspection of the model shows that the b-, c- and f-positions together form a contiguous rim with a convex shape. There are 11 such surface positions discernible in this alpha-helix. It was observed that, in principle, some flanking e-and g-positions might potentially aid in the formation of a contiguous binding surface, but this option was discarded in view of the risk to destabilize the Alphabodies and because the number of variable positions would run up too much. Thus, all 11 b-, c- and f-positions in the C-helix were initially considered for variegation, but the two N-terminal glutamates were finally left unaltered in order not to cancel out their capping function and to maintain the library completeness within reasonable bounds. This finally resulted in 9 b-, c- and f-positions to be variegated in the library. This library was accordingly termed 'scLib-C9'. The sequence is shown in Figure 3. This sequence is also provided as SEQ ID No: 4.

The actual single-chain Alphabody libraries were ordered at Geneart AG (Regensburg, Germany). A '3+3' monovalent display format (Smith, Gene 128:1-2 (1993)) was adopted using the pCx1 vector, a pHEN-derived phagemid. The libraries were delivered as transformed *E. coli* TG1 cells with a guaranteed minimum of 10⁸ unique clones. The Alphabody sequences were fused to the pIII coat protein of M13 phage. They were attached via their C-terminus to the pIII coat protein through a linker sequence that contains an amber codon (at the genetic level) and a His6-tag. Exportation of the fused Alphabodies to the periplasm was ensured by the presence of a PelB leader sequence at the N-terminus. The level of display on phage was checked using Western-Blotting and was found to be suitably high. Analysis showed that in general one third of the phage displayed an Alphabody (Figure 4).

### EXAMPLE 2. Alphabodies bindina to HIV-1 Env.

The present example demonstrates that single-chain Alphabodies can be obtained by a method, for example by using a mixture of Alphabody groove and helix .libraries as provided in EXAMPLE 1.

The viral fusion protein of interest was chosen to be HIV-1 Env. HIV-1 Env complexes, also known as 'envelope glycoprotein complexes' or 'gp120/gp41 complexes' or 'spikes', are a primary target for treatment of HIV infection. They are displayed at the surface of HIV virions and cells that are engineered so as to express Env spikes. HIV entry into a target cell and cell-cell fusion are primarily mediated by the action of these glycoprotein complexes subsequent to their engagement with specific receptors at the target cell. The ability to block viral entry or cellular fusion by impeding the function of Env complexes is generally thought to be of high value for the treatment of HIV infection. The reference sequence for HIV-1 Env is chosen to be that of the HXB2 strain; this sequence is also provided herein as SEQ ID No: 5 (MRVKEKYQHLWRWGWRWGTMLLGMLMICSATEKLWVTVYYGVPVWKEATTTLFCASDA KAYDTEVHNVWATHACVPTDPNPQEVVLVNVTENFNMWKNDMVEQMHEDIISLWDQSLK PCVKLTPLCVSLKCTDLKNDTNTNSSSGRMIMEKGEIKNCSFNISTSIRGKVQKEYAFFYKL DIIPIDNDTTSYKLTSCNTSVITQACPKVSFEPIPIHYCAPAGFAILKCNNKTFNGTGPCTNVS TVQCTHGIRPWSTQLLLNGSLAEEEWIRSVNFTDNAKTIIVQLNTSVEINCTRPNNNTRKRI RIQRGPGRAFVTIGKIGNMRQAHCNISRAKWNNTLKQIASKLREQFGNNKTIIFKQSSGGDP EIVTHSFNCGGEFFYCNSTQLFNSTWFNSTWSTEGSNNTEGSDTITLPCRIKQIINMWQKV GKAMYAPPISGQIRCSSNITGLLLTRDGGNSNNESEIFRPGGGDMRDNWRSELYKYKVVKI EPLGVAPTKAKRRWQREKRAVGIGALFLGFLGAAGSTMGAASMTLTVQARQLLSGIVQQ QNNLLRAI EAQQHLLQLTVWGIKQLQARILAVERYLKDQQLLGIWGCSGKLICTTAVPWNAS WSNKSLEQIWNHTTWMEWDREINNYTSLIHSLIEESQNQQEKNEQELLELDKWASLWNWF NITNWLWYIKLFIMIVGGLVGLRIVFAVLSIVNRVRQGYSPLSFQTHLPTPRGPDRPEGIEEE GGERDRDRSIRLVNGSLALIWDDLRSLCLFSYHRLRDLLLIVTRIVELLGRRGWEALKYWW NLLQYWSQELKNSAVSLLNATAIAVAEGTDRVIEVVQGACRAIRHIPRRIRQGLERILL).

The target region of interest within the said HIV-1 Env sequence was chosen to be the gp41 HR2 region, in particular, residues 628 to 661 of the HIV-1 HXB2 Env. The amino acid sequence is 'WMEWDREINNYTSLIHSLIEESQNQQEKNEQELLEL' (SEQ ID NO: 6) in single-letter notation, hereinafter also referred to as 'C36'. The target sequence (target peptide) used for the biopanning work described in the present example was a C36 derivative that was N-terminally biotinylated and C-terminally amidated and wherein the N-terminal biotin group was attached to the C36 sequence through a 4-residue Gly/Ser linker, the full target sequence having the amino acid sequence of SEQ ID No: 7, written in single-letter notation as 'biotin-GSGSWMEWDREINNYTSLIHSLIEESQNQQEKNEQELLEL-NH2', and herein also referred to as 'bL4_C36'.

A soluble biopanning protocol was applied to obtain (phage-displayed) Alphabodies which recognize the said bL4_C36 target, as follows. An equal mixture of phage displaying the libraries scLib_AC11, scLib_AC7 and scLib_C9 was prepared and used as input for the selection against the bL4_C36 target. The phage were incubated with the target for 1.5 to 2 hours and then captured on streptavidin magnetic beads for 15 to 30 minutes. Mock experiments where the target was omitted were always performed in parallel. Bound phage were eluted with an acidic pH shock after washing of the beads. Five selection rounds (biopanning rounds) were performed. The selection stringency, which was kept constant during the different rounds, was as follows: (i) amount of input phage: ~1.3 x 10¹² particles; (ii) 50 µl streptavidin-coated magnetic beads, target concentration: 500 nM; (iii) 10 washes (each with 1 ml 0.05% Tween 20-containing buffer).

In total, 162 phage colonies (39 from round 3, 32 from round 4 and 91 from round 5) were randomly picked and tested in phage ELISA, both on immobilized bL4_C36 and on an irrelevant control peptide. Phage were rescued in 96-well plates and tested without purification or quantification. None of the clones showed an absorbance (A₄₅₀ₙₘ) larger than 0.1 on the control peptide. Typically, phage clones showing an A₄₅₀ₙₘ larger than 10 times the average background signal were sequenced. 63 out of 66 clones were found to originate from the scLib_AC11 library, 3 originated from the scLib_AC7 library and none were found to come from the scLib_C9 library. The high abundance of binders from the groove libraries strongly suggests that Alphabodies preferably bind to the HR2 target sequence via a groove. Also, the higher frequency of binders from the scLib_AC11 library compared to the scLib_AC7 library suggests that the target sequence is ideally captured by an extended binding groove. The scLib_AC11 clones further separated into 12 distinct Alphabody sequences and the scLib_AC7 clones all showed the same sequence (data not shown).

Several of the positive Alphabody clones were selected for soluble expression. To this end, their coding sequences were subcloned into the pET16b vector (Novagen), in such way that they were appended with a N-terminal 10-histidine tag. The resulting constructs were transformed into a host *E. coli* strain harboring a chromosomal copy of the T7 polymerase gene under control of the lacUV5 promoter (DE3 lysogens), usually BL21(DE3). Transformed cells were grown in medium supplemented with ampicillin and protein expression was induced by the addition of IPTG to exponentially growing cultures. Cells containing the expressed Alphabodies were collected by centrifugation and the pellets were resuspended in 50 mM Tris, 500 mM NaCl, pH 7.8. Cells were then disrupted by sonication and spun down for cell debris removal. The cleared supernatants were applied onto a HITrap IMAC HP column (GE Healthcare) loaded with Ni²⁺ ions. Bound proteins were eluted by applying an imidazole gradient from 5 to 1000 mM. Alpha-body-containing fractions were pooled, concentrated and loaded on a Superdex 75 size exclusion chromatography (SEC) column (GE Healthcare). During this final purification step, the buffer was changed to 50 mM Tris, 150 mM NaCl, pH 7.8. Typically, the recovered protein fractions contained 0.3 to 2 mg/ml of pure Alphabody. Alphabody preparations can be stored for weeks at 4°C; for longer term storage, samples are kept at -80°C.

Figure 5 shows the results of an ELISA experiment on one of the purified anti-HR2 Alphabodies. Both the target peptide bL4_C36 and a biotinylated control peptide were immobilized on a neutravidin-coated and skim milk-blocked ELISA plate. The negative control peptide, referred to as 'bL4_N51', consisted of the HIV-1 Env HXB2 sequence of SEQ ID No: 1 residues 540 to 590 ('N51'), preceded by a 4-residue Gly-Ser-Gly-Ser linker ('L4') and an N-terminal biotin group ('b'). The Alphabody, herein referred to as 'scAB_Env03' (SEQ ID No: 8 and Figure 6), was subsequently applied to the wells at concentrations ranging from 0.4 µM to 0.4 nM and incubated for 2 h. Detection was performed with the Penta-His-HRP antibody (Qiagen). Significant binding was observed for the scAB_Env03 starting at concentrations of 1.5 to 3 nM; half saturation was obtained at about 6 nM and apparent maximal binding was reached at about 25 nM (Figure 5, black bars). No binding (A₄₅₀ₙₘ < 0.05) was observed to the negative control peptide bL4_N51 (white bars), or to peptide-free neutravidin (data not shown). This result shows that scAB_Env03 binds specifically to the target peptide bL4_C36, where the C36 part is an exact copy of HR2 in HIV-1 Env HXB2.

### EXAMPLE 3. Analysis of further HIV-1 Env-binding Alphabodies.

In addition to the scAB_Env03 Alphabody of EXAMPLE 2, three other single-chain Alphabodies, obtained from the same biopanning procedure, were further characterized. The present example demonstrates that multiple Alphabodies can be obtained, that their amino acid sequences can be determined, that they are highly thermostable, that they have a high affinity for HIV-1 Env, that their kinetics can be determined, and that some of them may be antivirally active.

The three additional Alphabodies that were tested are referred to as 'scAB_Env02', 'scAB_Env04' and 'scAB_Env05'. Their amino acid sequences are shown in Figure 7. Some apparently preferred amino acid residues were observed at different variegated positions, although not any position was occupied by a single, unique residue type. For example, three distinct Alphabodies had a proline at the first randomized position in the A-helix (i.e., at position g in the first heptad). The next randomized position seemed to prefer a small side chain with a hydroxyl group (i.e., serine or threonine). The fourth randomized position seemed to prefer a tryptophan. The C-helix showed a less conserved pattern, but some preference for hydrophobic residues was observed at the second, third and fourth randomized position.

Figure 8 shows a summary of thermal unfolding experiments as measured by circular dichroism (CD) at 222 nm in different concentrations of GuHCl denaturant. scAB_Env05 turned out to be the most stable, with a melting temperature (Tm) of about 100 °C even in 4 M GuHCl and a Tm = 61 °C at 6 M denaturant. scAB_Env03 was less stable by about 5-8 °C in Tm at 4-5 M GuHCl; this may be related to the presence of a proline at the first randomized position in the A-helix (see Figure 7), but yet this proline caused only a mild destabilization of the Alphabody structure. scAB_Env04 behaved somewhat aberrant, in that, a pre-transition was observed in the presence of GuHCl concentrations up to 4 M: upon unfolding, the CD signal decreased only a little, but with a Tm markedly lower than those of the other constructs. Then, at higher GuHCl concentrations, the full transition was observed with a Tm comparable to, though slightly less than, those of scAB_Env03 and scAB_Env05. This might be due to the presence of a glycine at the sixth variable position in helix A, as it is possible that the very last alpha-helical turn unfolds in advance of the rest of the scaffold. In conclusion, all constructs were found to be extremely stable and should be fully folded at room temperature, despite the occasional presence of proline and/or glycine in the alpha-helices.

ELISA experiments for scAB_Env02 to -05 showed very specific binding and a fairly high affinity for C36 (Figure 9). Especially the scAB_Env03 construct behaved most satisfactory: an onset of binding was observed in the low-nanomolar range, thereby confirming the results from EXAMPLE 2. The next strongest binders were scAB_Env02 and scAB_Env04, followed by scAB_Env05. The irrelevant control Alphabody scAB_PR02 (which was selected from an earlier biopanning procedure on an unrelated target) showed no background binding up to about 1 µM.

Three variants of the best-behaving scAB_Env03 Alphabody were synthesized according to the same protocol as the parental sequence. A first variant, denoted 'scAB_Env03_PA', was constructed wherein the proline at the first randomized position in the A-helix was replaced by an alanine (to test whether a helix-destabilizing proline could be substituted by a helix-stabilizing residue without loss of affinity). A second variant, denoted 'scAB_Env03_noM', was constructed wherein all methionines were replaced by either glycine or isoleucine (the methionines at the N-terminus of each helix were replaced by glycine, and those at the C-terminus of each helix were replaced by isoleucine). A third variant, denoted 'scAB_Env03_KC' was constructed wherein the lysine at the f-position in the second heptad of the B-helix was replaced by a cysteine to be able to test whether disulfide-linked or PEGylated Alphabodies retain their physical and biochemical properties. The sequences of these variants are shown in Figure 10.

Surface plasmon resonance (SPR) was used to determine the binding kinetics to immobilized bL4_C36 (Figure 11). All unmodified constructs (scAB_Env02 was not tested) showed Langmuir binding kinetics. The off-rate constants were in agreement with the ELISA results (scAB_Env03 < scAB_Env04 < scAB_Env05), confirming that scAB_Env03 was the most persistent binder. However the on-rate constants showed a different trend (scAB_Env04 > scAB_Env03 > scAB_Env05), resulting in about equal affinity for scAB_Env03 and scAB_Env04 (KD ≈ 200 nM) and a significant lower affinity for scAB_Env05 (KD ≈ 4000 nM). The Env03 Cys-mutant (scAB_Env03_KC) showed very strong binding: although the on-rate was lower than for the parental scAB_Env03 (kOn = 0.38 × 10⁵ M⁻¹ s⁻¹), the off-rate was extremely good (kOff = 2.4 x 10⁻⁵ s⁻¹), resulting in a subnanomolar affinity constant (KD = 0.62 nM). The main reason for this very high affinity is that Env03_KC dimerizes through the formation of a disulfide bond and binds in a bidentate mode onto the chip surface. This was confirmed in a later experiment (not shown) wherein the sample was pretreated with DTT and wherein the kinetics dropped back to about the same rates as the parental construct. The scAB_Env03_PA and scAB_Env03_noM constructs unexpectedly showed biphasic binding and dissociation kinetics. A first, fast association phase with on-rates comparable to the parental scAB_Env03 (i.e., kOn in the order of 10⁵ M⁻¹ s⁻¹) was followed by a second, slow phase (with kOn below 10⁻² s⁻¹). However, this second phase resulted in nearly persistent binding, with off-rate constants in the order of 10⁻⁴ s⁻¹. The global dissociation constants (KD = KD₁ * KD₂) were therefore close to about 10 nM for both constructs.

The HIV inhibitory properties of different Alphabodies were analyzed in a 5-day 'MTT infection assay' using a cell line (MT4-X4) displaying CD4 and CXCR4 receptors. The virus used in this assay was the laboratory adapted reference strain HXB2 virus using CXCR4 as co-receptor. Cells were infected with 100 TCID50/ml of virus in presence of three-fold dilutions of Alphabody starting at 2.5 µM. Inhibition of HIV infection by Alphabodies was evaluated by monitoring the cell survival using MTT (3-(4,5-Dimethylthiazol-2-YI)-2,5-Diphenyltetrazolium Bromide). MTT is reduced to formazan by living cells. Solubilization of the formazan crystals results in a colored product that can be measured by spectrophotometry at 540 nm. The cellular toxicity of the Alphabodies was monitored in the same assay using the same read-out, i.e., cell survival. As positive controls, the clinically approved T-20 peptide (Fuzeon®, Roche) was used as well as the CXCR4 antagonist AMD3100 (MozobileTM, Genzyme). Alphabodies scAB_Env03 and scAB_Env05 were tested using this assay, but showed no antiviral activity up to the highest concentration tested (2.5 µM). In contrast, the dimeric construct scAB_Env03_KC showed a clear antiviral activity with a 50% inhibitory concentration (IC50) of 709 nM (Figure 12). No toxicity effects were observed.

In conclusion, the present example demonstrates that Alphabodies can be obtained by a method of the present invention which display stable folding, high affinity to a subregion of a viral fusion protein, and significant antiviral activity on the virus displaying this viral fusion protein.

## Claims

1. A method for the production of at least one single-chain Alphabody polypeptide, having the general formula HRS1-L1-HRS2-L2-HRS3, wherein
- each of HRS1, HRS2 and HRS3 is independently a heptad repeat sequence (HRS) consisting of 2 to 7 consecutive heptad repeat units, at least 50% of all heptad a-and d-positions are occupied by isoleucine residues, each HRS starts and ends with an aliphatic or aromatic amino acid residue located at either a heptad a- or d-position, and HRS1, HRS2 and HRS3 together form a triple-stranded, alpha-helical, coiled coil structure; and
- each of L1 and L2 are independently a linker fragment, which covalently connects HRS1 to HRS2 and HRS2 to HRS3, respectively, and consisting of at least 4 amino acid residues, preferably at least 50% of which are selected from the group proline, glycine, serine; and
having detectable binding affinity for, or detectable in vitro activity on, a viral protein of interest, said method at least comprising the steps of:
a) producing a single-chain Alphabody library comprising at least 100 different-sequence single-chain Alphabody polypeptides, wherein said Alphabody polypeptides differ from each other in at least one of a defined set of 5 to 20 variegated amino acid residue positions, and wherein at least 70% of said variegated amino acid residue positions are located either:
(i) at heptad e-positions in a first alpha-helix of the Alphabody polypeptides and at heptad g-positions in a second alpha-helix, and optionally at heptad b-positions in said first alpha-helix of the Alphabody polypeptides and/or at heptad c-positions in said second alpha-helix of the Alphabody polypeptides, or
(ii) at heptad b-, c- and f-positions in one alpha-helix of the Alphabody polypeptides, or
(iii) at positions in a linker fragment connecting two consecutive alpha-helices of the Alphabody polypeptides, and
b) selecting from said single-chain Alphabody library at least one single-chain Alphabody having detectable binding affinity for, or detectable in vitro activity on, said viral protein of interest.

2. The method according to claim 1, wherein step a) of producing a single-chain Alphabody library comprises the steps of:
a1) producing a nucleic acid or vector library encoding a single-chain Alphabody library comprising at least 100 different-sequence single-chain Alphabody polypeptides, wherein said Alphabody polypeptides differ from each other in at least one of a defined set of 5 to 20 variegated amino acid residue positions, and wherein at least 70% of said variegated amino acid residue positions are located either:
(i) at heptad e-positions in a first alpha-helix of the Alphabody polypeptides and at heptad g-positions in a second alpha-helix, and optionally at heptad b-positions in said first alpha-helix of the Alphabody polypeptides and/or at heptad c-positions in said second alpha-helix of the Alphabody polypeptides, or
(ii) at heptad b-, c- and f-positions in one alpha-helix of the Alphabody polypeptides, or
(iii) at positions in a linker fragment connecting two consecutive alpha-helices of the Alphabody polypeptides, and
a2) expressing said nucleic acid or vector library under conditions suitable for the production of said single-chain Alphabody library.

3. The method according to claim 2, wherein the step a2) of expressing said nucleic acid or vector library, comprises introducing said nucleic acid or vector library into host cells and culturing said host cells in a medium under conditions suitable for the production of said single-chain Alphabody library.

4. The method according to claims 2 or 3, further comprising the step of isolating the single-chain Alphabody library produced in step a2) from said host cells and/or from said medium.

5. The method according to any one of claims 1 to 4, wherein step b) of selecting from said single-chain Alphabody library at least one single-chain Alphabody having detectable binding affinity for, or detectable in vitro activity on, said viral protein of interest, comprises contacting said viral protein of interest with the single-chain Alphabody library produced in step a).

6. The method according to any one of claims 1 to 5, further comprising the step of isolating from said single-chain Alphabody library said at least one single-chain Alphabody having detectable binding affinity for, or detectable in vitro activity on, said viral protein of interest.

7. The method according to any one of claims 1 to 6, wherein said Alphabody library is enriched for single-chain Alphabodies having detectable binding affinity for, or detectable in vitro activity on, said viral protein of interest.

8. The method according to claim 7, wherein said Alphabody library is enriched for single-chain Alphabodies having detectable binding affinity for, or detectable in vitro activity on, said viral protein of interest by iterative execution of step b) of selecting from said single-chain Alphabody library at least one single-chain Alphabody having detectable binding affinity for, or detectable in vitro activity on, said viral protein of interest.

9. The method according to any one of claims 1 to 8, further comprising the step of amplifying said at least one single-chain Alphabody having detectable binding affinity for, or detectable in vitro activity on, said viral protein of interest.

10. The method according to any one of claims 1 to 8, wherein the viral protein is a viral fusion protein.

11. The method according to any one of claims 1 to 10, wherein the viral protein is selected from the group consisting of the gp120 protein of HIV-1 virus, the HA1 and HA2 proteins of influenza, the F protein of respiratory syncytial virus, the HA protein of Influenza A virus, the HEF protein of influenza C virus, the F protein of Simian parainfluenza virus, the F protein of Human parainfluenza virus, the F protein of Newcastle disease virus, the F2 protein of measles, the F2 protein of Sendai virus, the gp2 protein of Ebola virus, the TM protein of Moloney murine leukemia virus, the gp41 protein of HIV-1, the gp41 protein of Simian immunodeficiency virus, the gp21 protein of Human T-cell leukemia virus 1, the TM protein of Human syncytin-2, the TM protein of Visna virus, the S2 protein of Mouse hepatitis virus, the E2 protein of SARS corona virus, the E protein of Tick-borne encephalitis virus, the E2 protein of Dengue 2 and 3 virus, the E protein of Yellow Fever virus, the E protein of West Nile virus, the E1 protein of Semliki forest virus, the E1 protein of Sindbis virus, the G protein of Rabies virus, the G protein of Vesicular stomatitis virus, the gB protein of Herpes simplex virus.

## Patentansprüche

1. Verfahren zur Herstellung wenigstens eines Einzelkette-Alphabody-Polypeptids mit der allgemeinen Formel HRS1-L1-HRS2-L2-HRS3, wobei
- es sich bei HRS1, HRS2 und HRS3 jeweils unabhängig um eine HRS (Heptad Repeat Sequence), die aus 2 bis 7 aufeinander folgenden Heptad-Repeat-Einheiten besteht, handelt, wenigstens 50% aller Heptad-Positionen a und d von Isoleucin-Resten besetzt sind, die HRS jeweils mit einem entweder an einer a- oder d-Heptad-Position lokalisierten aliphatischen oder aromatischen Aminosäurerest beginnt und endet und HRS1, HRS2 und HRS3 zusammen eine tripelsträngige, alpha-helikale Coiled-Coil-Struktur bilden; und
- es sich bei L1 und L2 jeweils unabhängig um ein Linker-Fragment handelt, das HRS1 mit HRS2 bzw. HRS2 mit HRS3 kovalent verbindet und aus wenigstens 4 Aminosäureresten besteht, von denen vorzugsweise wenigstens 50% aus der Gruppe Prolin, Glycin, Serin ausgewählt sind; und
mit nachweisbarer Bindungsaffinität für ein oder nachweisbarer In-vitro-Aktivität gegenüber einem Virusprotein von Interesse, wobei das Verfahren wenigstens die folgenden Schritte umfasst:
a) Herstellen einer Einzelkette-Alphabody-Bibliothek, die wenigstens 100 Einzelkette-Alphabody-Polypeptide mit unterschiedlicher Sequenz umfasst, wobei sich die Alphabody-Polypeptide voneinander in wenigstens einer von einem definierten Satz von 5 bis 20 variegierten Aminosäurerestpositionen unterscheiden und wobei wenigstens 70% der variegierten Aminosäurerestpositionen entweder
(i) an Heptad-Positionen e in einer ersten Alpha-Helix der Alphabody-Polypeptide und an Heptad-Positionen g in einer zweiten Alpha-Helix und gegebenenfalls an Heptad-Positionen b in der ersten Alpha-Helix der Alphabody-Polypeptide und/oder an Heptad-Positionen c in der zweiten Alpha-Helix der Alphabody-Polypeptide oder
(ii) an Heptad-Positionen b, c und f in einer Alpha-Helix der Alphabody-Polypeptide oder
(iii) an Positionen in einem zwei aufeinander folgende Alpha-Helices der Alphabody-Polypeptide verbindenden Linker-Fragment lokalisiert sind, und
b) Auswählen wenigstens eines Einzelkette-Alphabody mit nachweisbarer Bindungsaffinität für das oder nachweisbarer In-vitro-Aktivität gegenüber dem Virusprotein von Interesse aus der Einzelkette-Alphabody-Bibliothek.

2. Verfahren nach Anspruch 1, wobei Schritt a) der Herstellung einer Einzelkette-Alphabody-Bibliothek die folgenden Schritte umfasst:
a1) Herstellen einer Nukleinsäure- oder Vektorbibliothek, die eine Einzelkette-Alphabody-Bibliothek codiert, die wenigstens 100 Einzelkette-Alphabody-Polypeptide mit unterschiedlicher Sequenz umfasst, wobei sich die Alphabody-Polypeptide voneinander in wenigstens einer von einem definierten Satz von 5 bis 20 variegierten Aminosäurerestpositionen unterscheiden und wobei wenigstens 70% der variegierten Aminosäurerestpositionen entweder
(i) an Heptad-Positionen e in einer ersten Alpha-Helix der Alphabody-Polypeptide und an Heptad-Positionen g in einer zweiten Alpha-Helix und gegebenenfalls an Heptad-Positionen b in der ersten Alpha-Helix der Alphabody-Polypeptide und/oder an Heptad-Positionen c in der zweiten Alpha-Helix der Alphabody-Polypeptide oder
(ii) an Heptad-Positionen b, c und f in einer Alpha-Helix der Alphabody-Polypeptide oder
(iii) an Positionen in einem zwei aufeinander folgende Alpha-Helices der Alphabody-Polypeptide verbindenden Linker-Fragment lokalisiert sind, und
a2) Exprimieren der Nukleinsäure- oder Vektorbibliothek unter für die Herstellung der Einzelkette-Alphabody-Bibliothek geeigneten Bedingungen.

3. Verfahren nach Anspruch 2, wobei der Schritt a2) des Exprimierens der Nukleinsäure- oder Vektorbibliothek Einführen der Nukleinsäure- oder Vektorbibliothek in Wirtszellen und Kultivieren der Wirtszellen in einem Medium unter für die Herstellung der Einzelkette-Alphabody-Bibliothek geeigneten Bedingungen umfasst.

4. Verfahren nach Anspruch 2 oder 3, ferner umfassend den Schritt des Isolierens der in Schritt a2) hergestellten Einzelkette-Alphabody-Bibliothek aus den Wirtszellen und/oder aus dem Medium.

5. Verfahren nach einem der Ansprüche 1 bis 4, wobei Schritt b) des Auswählens wenigstens eines Einzelkette-Alphabody mit nachweisbarer Bindungsaffinität für das oder nachweisbarer In-vitro-Aktivität gegenüber dem Virusprotein von Interesse aus der Einzelkette-Alphabody-Bibliothek Inkontaktbringen des Virusproteins von Interesse mit der in Schritt a) hergestellten Einzelkette-Alphabody-Bibliothek umfasst.

6. Verfahren nach einem der Ansprüche 1 bis 5, ferner umfassend den Schritt des Isolierens des wenigstens einen Einzelkette-Alphabody mit nachweisbarer Bindungsaffinität für das oder nachweisbarer In-vitro-Aktivität gegenüber dem Virusprotein von Interesse aus der Einzelkette-Alphabody-Bibliothek.

7. Verfahren nach einem der Ansprüche 1 bis 6, wobei die Alphabody-Bibliothek für Einzelkette-Alphabodies mit nachweisbarer Bindungsaffinität für das oder nachweisbarer In-vitro-Aktivität gegenüber dem Virusprotein von Interesse angereichert wird.

8. Verfahren nach Anspruch 7, wobei die Alphabody-Bibliothek für Einzelkette-Alphabodies mit nachweisbarer Bindungsaffinität für das oder nachweisbarer In-vitro-Aktivität gegenüber dem Virusprotein von Interesse durch iterative Ausführung von Schritt b) des Auswählens wenigstens eines Einzelkette-Alphabody mit nachweisbarer Bindungsaffinität für das oder nachweisbarer In-vitro-Aktivität gegenüber dem Virusprotein von Interesse aus der Einzelkette-Alphabody-Bibliothek angereichert wird.

9. Verfahren nach einem der Ansprüche 1 bis 8, ferner umfassend den Schritt des Amplifizierens des wenigstens einen Einzelkette-Alphabody mit nachweisbarer Bindungsaffinität für das oder nachweisbarer In-vitro-Aktivität gegenüber dem Virusprotein von Interesse.

10. Verfahren nach einem der Ansprüche 1 bis 8, wobei es sich bei dem Virusprotein um ein Virusfusionsprotein handelt.

11. Verfahren nach einem der Ansprüche 1 bis 10, wobei das Virusprotein aus der aus dem gpl20-Protein von HIV-1-Virus, den Influenza-Proteinen HA1 und HA2, dem F-Protein von RSV (Respiratory Syncytial Virus), dem HA-Protein von Influenza-A-Virus, dem HEF-Protein von Influenza-C-Virus, dem F-Protein von SPIV (Simian Parainfluenza Virus), dem F-Protein von HPIV (Human Parainfluenza Virus), dem F-Protein von Newcastle-Krankheit-Virus, dem Masern-F2-Protein, dem F2-Protein von Sendai-Virus, dem gp2-Protein von Ebola-Virus, dem TM-Protein von MMLV (Moloney Murine Leukemia Virus), dem gp41-Protein von HIV-1, dem gp41-Protein von SIV (Simian Immunodeficiency Virus), dem gp21-Protein von HTLV-1 (Human T-cell Leukemia Virus 1), dem TM-Protein von Human-Syncytin-2, dem TM-Protein von Visna-Virus, dem S2-Protein von Maus-Hepatitis-Virus, dem E2-Protein von SARS-Coronavirus, dem E-Protein von FSME-Virus (Tick-borne Encephalitis Virus), dem E2-Protein von Dengue-2-und -3-Virus, dem E-Protein von Gelbfieber-Virus, dem E-Protein von West-Nil-Virus, dem E1-Protein von SFV (Semliki Forest Virus), dem E1-Protein von Sindbis-Virus, dem G-Protein des Tollwutvirus, dem G-Protein von VSV (Vesicular Stomatitis Virus), dem gB-Protein von Herpes-simplex-Virus bestehenden Gruppe ausgewählt ist.

## Revendications

1. Procédé de production d'au moins un polypeptide d'Alphabody monocaténaire, ayant la formule générale HRS1-L1-HRS2-L2-HRS3, dans laquelle
- chacun de HRS1, HRS2 et HRS3 est indépendamment une séquence de répétition d'heptade (HRS) constituée de 2 à 7 motifs de répétition d'heptade consécutifs, au moins 50 % de toutes les positions d'heptade a et d sont occupées par des résidus isoleucine, chaque HRS commence et termine par un résidu d'acide aminé aliphatique ou aromatique situé à une position d'heptade a ou d, et HRS1, HRS2 et HRS3 forment conjointement une structure de superhélice, de type hélice alpha, tricaténaire ; et
- chacun de L1 et L2 est indépendamment un fragment de lieur, qui relie de façon covalente HRS1 à HRS2 et HRS2 à HRS3, respectivement, et constitué d'au moins 4 résidus d'acide aminé, de préférence dont au moins 50 % sont choisis dans le groupe de la proline, la glycine, sérine ; et
ayant une affinité de liaison détectable pour, ou une activité *in vitro* détectable sur, une protéine virale d'intérêt, ledit procédé comprenant au moins les étapes de :
a) production d'une banque d'Alphabody monocaténaires comprenant au moins 100 polypeptides d'Alphabody monocaténaires de séquences différentes, lesdits polypeptides d'Alphabody différant les uns des autres à au moins une d'un ensemble défini de 5 à 20 positions de résidu d'acide aminé variées, et au moins 70 % desdites positions de résidu d'acide aminé variées étant situées soit :
(i) aux positions d'heptade e dans une première hélice alpha des polypeptides d'Alphabody et aux positions d'heptade g dans une deuxième hélice alpha, et facultativement aux positions d'heptade b dans ladite première hélice alpha des polypeptides d'Alphabody et/ou aux positions d'heptade c dans ladite deuxième hélice alpha des polypeptides d'Alphabody, ou
(ii) aux positions d'heptade b, c et f dans une hélice alpha des polypeptides d'Alphabody, ou
(iii) aux positions dans un fragment de lieur reliant deux hélices alpha consécutives des polypeptides d'Alphabody, et
b) sélection dans ladite banque d'Alphabody monocaténaires d'au moins un Alphabody monocaténaire ayant une affinité de liaison détectable pour, ou activité *in vitro* détectable sur, ladite protéine virale d'intérêt.

2. Procédé selon la revendication 1, dans lequel l'étape a) de production d'une banque d'Alphabody monocaténaires comprend les étapes de :
a1) production d'une banque d'acide nucléiques ou de vecteurs codant pour une banque d'Alphabody monocaténaires comprenant au moins 100 polypeptides d'Alphabody monocaténaires de séquences différentes, lesdits polypeptides d'Alphabody différant les uns des autres à au moins une d'un ensemble défini de 5 à 20 positions de résidu d'acide aminé variées, et au moins 70 % desdites positions de résidu d'acide aminé variées étant situées soit :
(i) aux positions d'heptade e dans une première hélice alpha des polypeptides d'Alphabody et aux positions d'heptade g dans une deuxième hélice alpha, et facultativement aux positions d'heptade b dans ladite première hélice alpha des polypeptides d'Alphabody et/ou aux positions d'heptade c dans ladite deuxième hélice alpha des polypeptides d'Alphabody, ou
(ii) aux positions d'heptade b, c et f dans une hélice alpha des polypeptides d'Alphabody, ou
(iii) aux positions dans un fragment de lieur reliant deux hélices alpha consécutives des polypeptides d'Alphabody, et
a2) l'expression de ladite banque d'acides nucléiques ou de vecteurs dans des conditions adaptées pour la production de ladite banque d'Alphabody monocaténaires.

3. Procédé selon la revendication 2, dans lequel l'étape a2) d'expression de ladite banque d'acides nucléiques ou de vecteurs, comprend l'introduction de ladite banque d'acides nucléiques ou de vecteurs dans des cellules hôtes et culture desdites cellules hôtes dans un milieu dans des conditions adaptées pour la production de ladite banque d'Alphabody monocaténaires.

4. Procédé selon les revendications 2 ou 3, comprenant en outre l'étape d'isolement de la banque d'Alphabody monocaténaires produite dans l'étape a2) à partir desdites cellules hôtes et/ou dudit milieu.

5. Procédé selon l'une quelconque des revendications 1 à 4, dans lequel l'étape b) de sélection à partir de ladite banque d'Alphabody monocaténaires d'au moins un Alphabody monocaténaire ayant une affinité de liaison détectable pour, ou une activité *in vitro* détectable sur, ladite protéine virale d'intérêt, comprend la mise en contact de ladite protéine virale d'intérêt avec la banque d'Alphabody monocaténaires produite dans l'étape a).

6. Procédé selon l'une quelconque des revendications 1 à 5, comprenant en outre l'étape d'isolement à partir de ladite banque d'Alphabody monocaténaires dudit au moins un Alphabody monocaténaire ayant une affinité de liaison détectable pour, ou une activité *in vitro* détectable sur, ladite protéine virale d'intérêt.

7. Procédé selon l'une quelconque des revendications 1 à 6, dans lequel ladite banque d'Alphabody est enrichi en Alphabody monocaténaires ayant une affinité de liaison détectable pour, ou une activité *in vitro* détectable sur, ladite protéine virale d'intérêt.

8. Procédé selon la revendication 7, dans lequel ladite banque d'Alphabody est enrichie en Alphabody monocaténaires ayant une affinité de liaison détectable pour, ou une activité *in vitro* détectable sur, ladite protéine virale d'intérêt par exécution itérative de l'étape b) de sélection à partir de ladite banque d'Alphabody monocaténaires d'au moins un Alphabody monocaténaire ayant une affinité de liaison détectable pour, ou une activité *in vitro* détectable sur, ladite protéine virale d'intérêt.

9. Procédé selon l'une quelconque des revendications 1 à 8, comprenant en outre l'étape d'amplification dudit au moins un Alphabody monocaténaire ayant une affinité de liaison détectable pour, ou une activité *in vitro* détectable sur, ladite protéine virale d'intérêt.

10. Procédé selon l'une quelconque des revendications 1 à 8, dans lequel la protéine virale est une protéine de fusion virale.

11. Procédé selon l'une quelconque des revendications 1 à 10, dans lequel la protéine virale est choisie dans le groupe constitué de la protéine gp120 du virus VIH-1, les protéines HA1 et HA2 de la grippe, la protéine F du virus syncytial respiratoire, la protéine HA du virus de la grippe A, la protéine HEF du virus de la grippe C, la protéine F du virus parainfluenza simien, la protéine F du virus parainfluenza humain, la protéine F du virus de la maladie de Newcastle, la protéine F2 de la rougeole, la protéine F2 du virus Sendai, la protéine gp2 du virus Ebola, la protéine TM du virus de la leucémie murine de Moloney, la protéine gp41 de VIH-1, la protéine gp41 du virus d'immunodéficience simien, la protéine gp21 du virus de la leucémie à lymphocytes T humaine 1, la protéine TM de syncytine 2 humaine, la protéine TM du virus Visna, la protéine S2 du virus de l'hépatite de la souris, la protéine E2 du SARS coronavirus, la protéine E du virus de l'encéphalite à tiques, la protéine E2 du virus de la dengue 2 et 3, la protéine E du virus de la fièvre jaune, la protéine E du virus du Nil occidental, la protéine E1 du virus de la forêt de Semliki, la protéine E1 du virus Sindbis, la protéine G du virus de la rage, la protéine G du virus de la stomatite vésiculaire et la protéine gB du virus de l'herpès simplex.
